(19) Europäisches Patentamt
European Patent Office
Office européen des brevets

(11) Publication number: **0 071 544**
**B1**

(12) # EUROPEAN PATENT SPECIFICATION

(45) Date of publication of patent specification: **08.10.86**

(21) Application number: **82401459.1**

(22) Date of filing: **03.08.82**

(51) Int. Cl.[4]: **C 07 F 9/65**, C 07 F 9/44, C 07 K 5/06, A 61 K 31/66

(54) **Phosphonamidate compounds.**

(30) Priority: **03.08.81 US 289671**

(43) Date of publication of application: **09.02.83 Bulletin 83/06**

(45) Publication of the grant of the patent: **08.10.86 Bulletin 86/41**

(84) Designated Contracting States: **AT BE CH DE FR GB IT LI LU NL SE**

(56) References cited:
**EP-A-0 009 183**
**EP-A-0 058 427**

**Proceedings of the National Academy of Science (USA), Volume 76, pages 6216 to 6220, 1979**

**PROCEEDINGS OF THE NATIONAL ACADEMY OF SCIENCES OF THE UNITED STATES OF AMERICA, vol. 79, no. 7, (April 1982) E.D. THORSETT et al.: "Phosphorus containing inhibitors of angiotensinconverting enzyme"**

(73) Proprietor: **E.R. Squibb & Sons, Inc.**
**Lawrenceville-Princeton Road**
**Princeton, N.J. 08540 (US)**

(72) Inventor: **Karanewski, Donald S.**
**511 Village Road West**
**Princeton Junction New Jersey (US)**
Inventor: **Petrillo, Edward W.**
**433 Sked Street**
**Pennington New Jersey (US)**

(74) Representative: **Maiffret, Bernard et al**
**Law Offices of William J. Rezac**
**49 avenue Franklin D. Roosevelt**
**F-75008 Paris (FR)**

(56) References cited:
**JOURNAL OF MEDICINAL CHEMISTRY, vol. 24, no. 4 (April 1981) M.A. ONDETTI et al.: "Inhibition of the Renin-Angiotensin System. A New Approach to the Therapy of Hypertension", pages 355-361**

**EP 0071 544 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
CHEMICAL ABSTRACTS, vol. 94, no. 5, (02-02-1981), ref. 26702w page 218 Columbus, Ohio, US R.E. GALARDY et al.: "Inhibition of angiotensin-converting enzyme with N alpha-phosphoryl-l-alanyl-l-proline and N alpha-phosphoryl-l-valyl-L-tryptophan"

**Description**

This invention in its broadest aspects relates to the phosphonamidate substituted imino or amino acid compounds of formula I below, and to compositions containing such compounds as anti-hypertensive agents. More specifically, this invention is directed to new phosphonamidate substituted amino or imino acids of formula I and pharmaceutically acceptable salts thereof

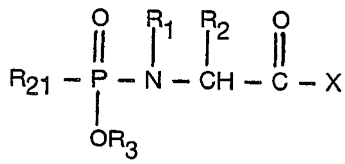

(I)

wherein:

X is 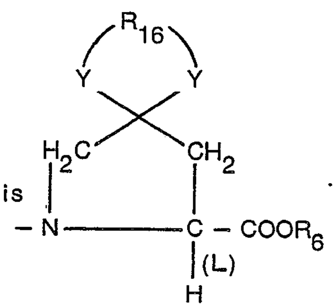 or 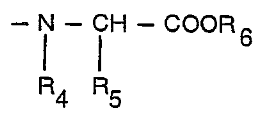 ;

Y is oxygen or sulfur.

$R_{16}$ is an ethylene or trimethylene radical in which one or more of the carbon atoms may have an alkyl of 1 to 4 carbon atoms or a di(alkyl of 1 to 4 carbon atoms) substituent.

$R_4$ is hydrogen, lower alkyl, $-(CH_2)_m-$ cycloalkyl, or

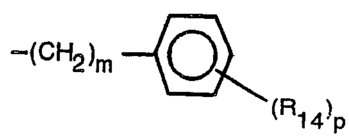

$R_5$ is hydrogen, lower alkyl,

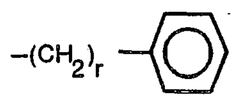 , 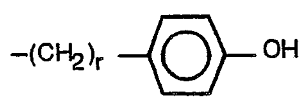 ,

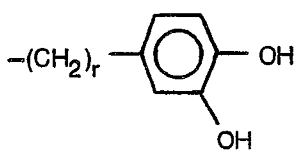 , 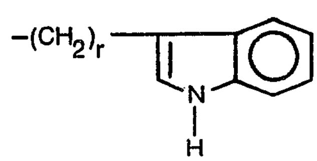 ,

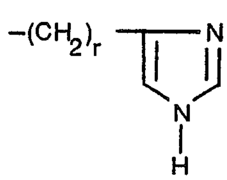 , $-(CH_2)_r-NH_2$ , $-(CH_2)_r-SH$ ,

$-(CH_2)_r-S-$lower alkyl , 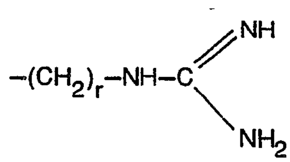 ,

or 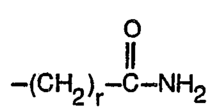 .

r is an integer from 1 to 4.
$R_1$ is hydrogen, lower alkyl, or cycloalkyl.
$R_2$ is hydrogen, lower alkyl, halo substituted lower alkyl,

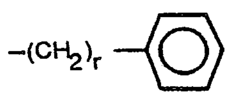 , 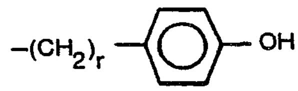 , 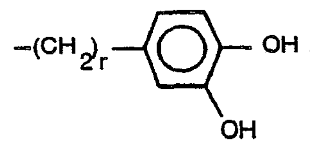 ,

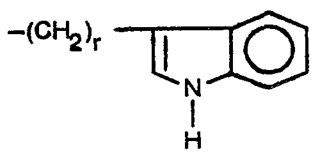 , 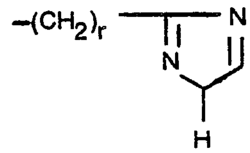 ,

$-(CH_2)_r-NH_2$ , $-(CH_2)_r-SH$ , $-(CH_2)_r-S-$lower alkyl ,

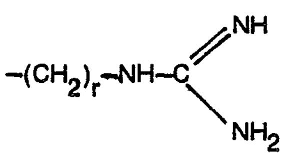 , 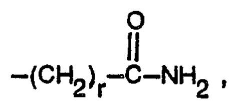 ,

or $R_1$ and $R_2$ taken together are $-(CH_2)_n-$ wherein n is an integer from 2 to 4.

$R_3$ and $R_6$ are independently selected from hydrogen, alkali metal, lower alkyl, benzyl, benzhydryl, or

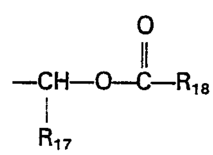

wherein $R_{17}$ is hydrogen, lower alkyl, cycloalkyl, or phenyl, and $R_{18}$ is hydrogen, lower alkyl, lower alkoxy, phenyl, or $R_{17}$ and $R_{18}$ taken together are $-(CH_2)_2-$, $-(CH_2)_3-$, $-CH{=}CH-$, or

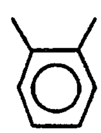 .

$R_{21}$ is alkyl of 1 to 10 carbons,

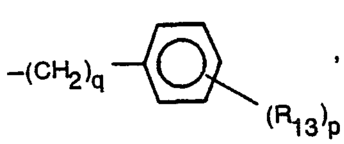 , $-(CH_2)_q-$cycloalkyl , $-(CH_2)_s-NH_2$ ,

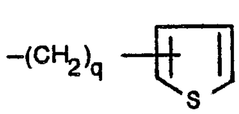 , 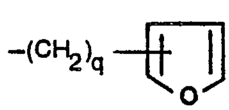 , or 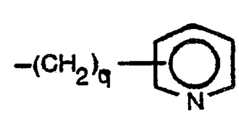

wherein q is zero or an integer from 1 to 7, s is an integer from 1 to 8;

$R_{13}$ is hydrogen, alkyl of 1 to 4 carbons, alkoxy of 1 to 4 carbons, alkylthio of 1 to 4 carbons, chloro, bromo, fluoro, trifluoromethyl, hydroxy, phenyl, phenoxy, phenylthio, or phenylmethyl;

$R_{14}$ is hydrogen, lower alkyl of 1 to 4 carbons, alkoxy of 1 to 4 carbons, alkylthio of 1 to 4 carbons, chloro, bromo, fluoro, trifluoromethyl, or hydroxy;

m is zero, one, two or three; and

p is one, two or three provided that p is more than one only if $R_{13}$ or $R_{14}$ is hydrogen, methyl, methoxy, chloro, or fluoro.

The term alkyl used in defining $R_{21}$ refers to straight or branched chain saturated hydrocarbon radicals having up to ten carbons, for example, methyl, ethyl, propyl, isopropyl, butyl, isobutyl, t-butyl, pentyl, isopentyl, heptyl, octyl, decyl. The term lower alkyl used in defining various symbols refers to straight or branched chain radicals having up to seven carbons. The preferred lower alkyl groups are up to four carbons with methyl and ethyl most preferred. Similarly the terms lower alkoxy and lower alkylthio refer to such lower alkyl groups attached to an oxygen or sulfur.

The term cycloalkyl refers to saturated rings of 3 to 7 carbon atoms with cyclopentyl and cyclohexyl being most preferred.

The term halo substituted lower alkyl refers to such lower alkyl groups described above in which one or more hydrogens have been replaced by chloro, bromo or fluoro groups such as trifluoromethyl, which is preferred, pentafluoroethyl, 2,2,2-trichloroethyl, chloromethyl, bromomethyl.

The symbols

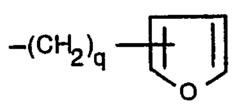 , 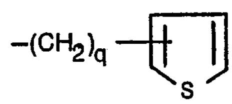 , and 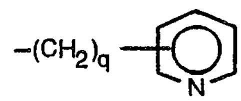

represent that the alkylene bridge is attached to an available carbon atom.

Thorsett, et al. in European Patent Application Serial No. 9,183 disclose phosphoryl derivatives of aminoacids including proline. These compounds are disclosed as being hypotensive agents due to their angiotensin converting enzyme inhibition activity.

Petrillo in United States Patent 4,168,267 discloses that various phosphinylalkanoyl substituted prolines are useful as hypotensive agents due to their ability to inhibit the angiotensin converting enzyme.

Ondetti, et al. in United States Patent 4,151,172 discloses that various phosphonoacyl prolines are useful as hypotensive agents due to their ability to inhibit the angiotensin converting enzyme.

Mercaptoacyl derivatives of proline and substituted prolines are known to be useful hypotensive agents due to their angiotensin converting enzyme inhibition activity.

Ondetti, et al. in U.S. Patent 4,105,776 disclose such compounds wherein the proline ring is unsubstituted or substituted by an alkyl or hydroxy group. Ondetti, et al. in U.S. Patent 4,154,935 disclose such compounds wherein the proline ring is substituted with one or more halogens. Ondetti, et al. in U.K. Patent Application 2,028,327 and U.S. Patent 4,316,906 disclose such compounds wherein the proline ring is substituted by various ethers and thioethers. Krapcho in U.S. Patent 4,217,359 disclose such compounds wherein the proline ring has a carbamoyloxy substituent. Krapcho in U.K. Patent Application 2,039,478 and U.S. Patent 4,311,697 disclose compounds wherein the proline ring has a diether, dithioether, ketal or thioketal substituent in the 4-position. Krapcho in U.S. Patent 4,316,905, discloses such compounds wherein the proline ring has a cycloalkyl, phenyl, or phenyl-lower alkylene substituent. Ondetti, et al. in U.S. Patent 4,234,489 disclose such compounds wherein the proline has a keto substituent in the 5-position. Krapcho, et al. in U.S. Serial No. 162,341 filed June 23, 1980 disclose such compounds wherein the proline has an imido, amido, or amino substituent in the 4-position. Iwao, et al. in U.K. Patent Application 2,027,025 disclose such compounds wherein the proline has an aromatic substituent in the 5-position. Ondetti, et al. in U.S. Patents 4,053,651 and 4,199,512 disclose that mercaptoacyl derivatives of various aminoacids other than proline are also useful angiotensin converting enzyme inhibitors.

Mercaptoacyl derivatives of 3,4-dihydroproline are disclosed as angiotensin converting enzyme inhibitors by Ondetti in U.S. Patent 4,129,566. Mercaptoacyl derivatives of thiazolidinecarboxylic acid and substituted thiazolidinecarboxylic acid are disclosed as angiotensin converting enzyme inhibitors by Ondetti in U.S. Patent 4,192,878 and by Yoshitomo Pharmaceutical Ind. in Belgian Patent 868,532.

Mercaptoacyl tetrahydroisoquinoline compounds are disclosed by Portlock in U.K. Application 2,048,863 and by Hayashi et al, in U.S. Patent 4,256,751.

EP—A—0 058 427 filed February 16, 1982 discloses substituted phosphonamides useful as converting enzyme inhibitors and as antihypertensives. However, this prior application was published on August 25, 1982, i.e. after the date of filing of the present application.

The compounds of formula I wherein $R_{21}$ is other than $—(CH_2)_s—NH_2$ are prepared according to the following procedures. A phosphonochloridate of formula II wherein $R_3$ is lower alkyl, benzyl or benzhydryl

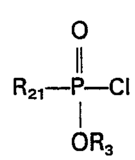 (II)

is coupled with a peptide ester of the formula

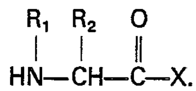

(III)

Preferably, the peptide ester of formula III is in its hydrochloride salt form and $R_6$ in the definition of X is lower alkyl, benzyl or benzhydryl.

These compounds of formula I can also be prepared by coupling an acid or its activated form of formula IV wherein $R_3$ is lower alkyl, benzyl or benzhydryl

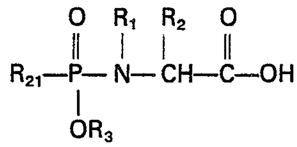

(IV)

with an imino or amino acid or ester of the formula

HX. (V)

The term activated form refers to the conversion of the acid to a mixed anhydride, symmetrical anhydride, acid chloride, or activated ester, see Methoden der Organischen Chemie (Houben-Weyl), Vol. XV, part II, page 1 et seq. (1974) for a review of the methods of acylation. Preferably the reaction is performed in the presence of a coupling agent such as 1,1-carbonyldiimidazole, thionyl chloride, or dicyclohexylcarbodiimide.

In the above reactions if $R_5$ and/or $R_2$ is

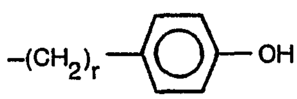 , 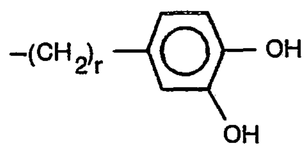 , $-(CH_2)_r-NH_2$ ,

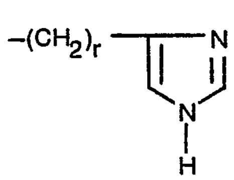 , $-(CH_2)_r-SH$ , or 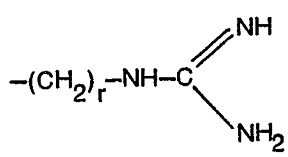

then the hydroxy, amino, imidazolyl, mercaptan, or guanidinyl function should be protected during the coupling reaction. Suitable protecting groups include benzyloxycarbonyl, t-butoxycarbony, benzyl, benzhydryl, trityl, and nitro in the case of guanidinyl. The protecting group is removed by hydrogenation, treatment with acid, or other known methods following completion of the reaction.

The products of formula I wherein either or both of $R_3$ and $R_6$ are lower alkyl, benzyl, or benzhydryl can be hydrogenated, for example, by treating with hydrogen in the presence of a palladium on carbon catalyst or chemically treated such as with sodium hydroxide in aqueous dioxane or with trimethylsilylbromide in dichloromethane to yield the products of formula I wherein $R_3$ and $R_6$ are hydrogen.

The ester products of formula I wherein $R_6$ is

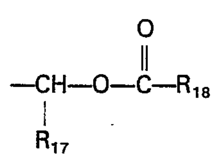

may be obtained by employing the peptide of formula III or the imino or amino acid of formula V in the above reactions with the ester group already in place. Such ester reactants can be prepared by treating peptide, imino, or amino acids with an acid chloride such as

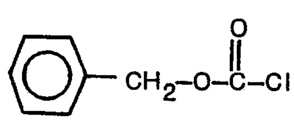 or 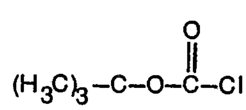

so as to protect the N-atom. The protected acid compound is then reacted in the presence of base with a compound of the formula

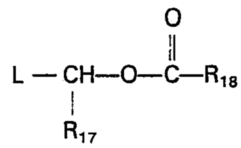

(VI)

wherein L is a leaving group such as chlorine, bromine, tolylsulfonyloxy, etc., followed by removal of the N-protecting group such as by treatment with acid or hydrogenation.

The ester products of formula I wherein $R_6$ is

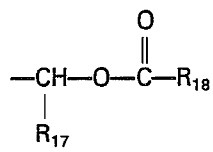

can also be obtained by treating the product of formula I wherein $R_6$ is hydrogen with a molar equivalent of the compound of formula VI. The diester products wherein $R_3$ and $R_6$ are the same and are

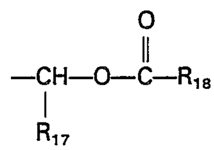

can be obtained by treating the product of formula I wherein $R_3$ and $R_6$ are both hydrogen or an alkali metal with two or more equivalents of the compound of formula VI.

The ester products of formula I wherein $R_3$ is

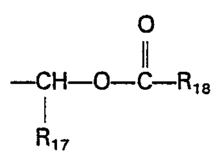

can be obtained by treating the product of formula I wherein $R_3$ is hydrogen or an alkali metal and $R_6$ is benzyl or benzhydryl with the compound of formula VI in the presence of base. Removal of the $R_6$ ester group such as by hydrogenation yields the products of formula I wherein $R_3$ is

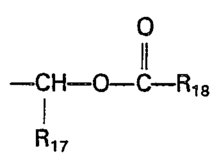

and $R_6$ is hydrogen.

The phosphonamidate reactants of formula IV can be prepared by coupling the phosphonochloridate of formula II wherein $R_3$ is lower alkyl, benzyl, or benzhydryl with the amino acid ester such as the benzyl or benzhydryl ester of the formula

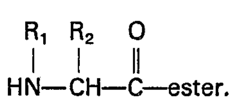

(VII)

The phosphonochloridates of formula II are described in the literature and in particular by Kosolapoff, et al. in Organic Phosphorous Compounds, Vol. 7, Chapter 18 (Wiley 1972).

The various peptides of formula III and imino and amino acids and esters of formula V are described in the literature and in the various patents referred to above. Various substituted prolines are also disclosed by Mauger et al., Chem. Review, Vol. 66, p. 47—86 (1966). When the amino or imino acid is known, it can be readily converted to the ester by conventional means. For example, the esters where $R_6$ is t-butyl can be obtained by treating the corresponding N-carbobenzyloxyimino acid with isobutylene under acidic conditions and then removing the N-carbobenzyloxy protecting group by catalytic hydrogenation and the

esters wherein $R_6$ is benzyl can be obtained by treating the imino acid with benzyl alcohol and thionyl chloride.

The compounds of formula I wherein $R_{21}$ is $-(CH_2)_s-NH_2$ are prepared by reacting a phthalidyl protected compound of the formula

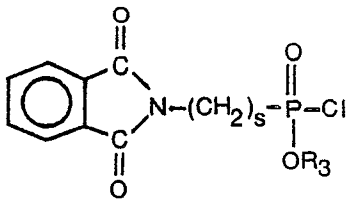

(XII)

wherein $R_3$ is lower alkyl, benzyl, or benzhydryl with the peptide ester of formula III, preferably wherein $R_6$ in the definitiion of X is benzyl, in the presence of triethylamine to yield the protected compound of formula

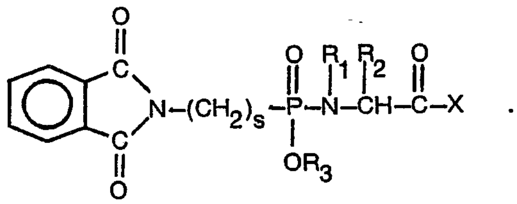

(XIII)

Treatment with hydrazine removes the phthalidyl protecting group after which the $R_3$ and $R_6$ ester group can be removed as described previously to yield the corresponding diacid compounds of formula I.

The phosphonochloridates of formula XII can be prepared by treating a phthalidyl protected alkylbromide of the formula

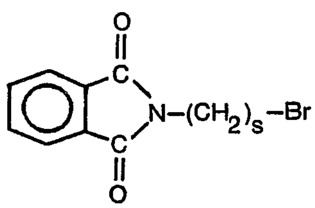

(XIV)

with a trialkylphosphite of the formula

$$P(O\text{-alkyl})_3 \quad \text{(XV)}$$

to yield the diester of the formula

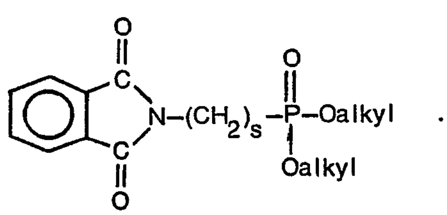

(XVI)

Treatment of this diester with trimethylsilylbromide yields the phosphonic acid of the formula

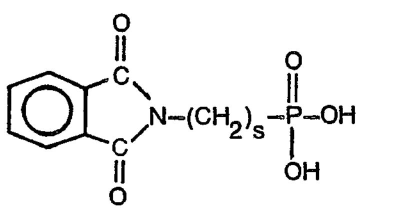

(XVII)

The acid of formula XVII can then be treated with phosphorus pentachloride and an alcohol of the formula

$$R_3—OH \qquad (XVIII)$$

in the presence of triethylamine to give the comound of formula XII.

Preferred compounds of this invention with respect to the amino or imino acid or ester part of the structure of formula I are those wherein:

$R_4$ is hydrogen, methyl, cyclohexyl, phenyl or benzyl.

$R_5$ is hydrogen, alkyl of 1 to 4 carbons,

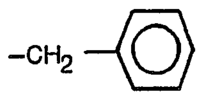 , 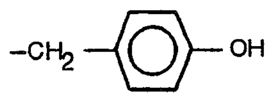 , 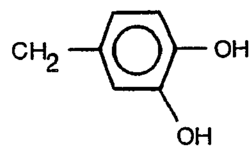 ,

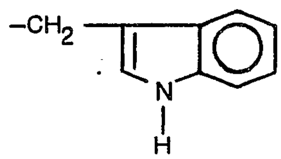 , 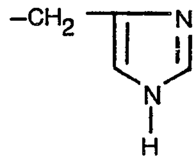 , $-(CH_2)_4-NH_2$ , $-CH_2SH$ ,

$-(CH_2)_2-S-CH_3$ , 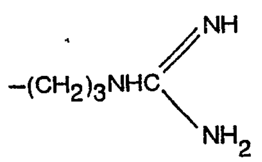 , 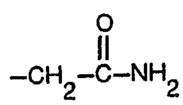 , or 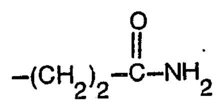 .

$R_6$ is hydrogen, an alkali metal, or

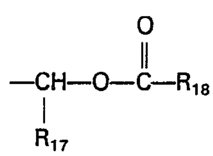

$R_{17}$ is hydrogen, straight or branched chain lower alkyl of 1 to 4 carbons, or cyclohexyl and $R_{18}$ is straight or branched chain lower alkyl of 1 to 4 carbons or phenyl.

Y is O or S, $R_{16}$ is an ethylene or trimethylene radical in which one or more of the carbons has a methyl or dimethyl substituent.

Most preferred compounds of this invention with respect to the amino or imino acid or ester part of the structure of formula I are those wherein:

X is 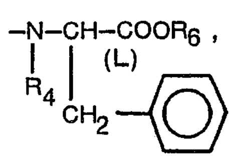 , 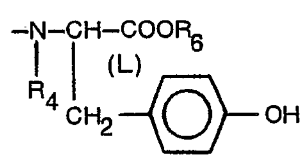

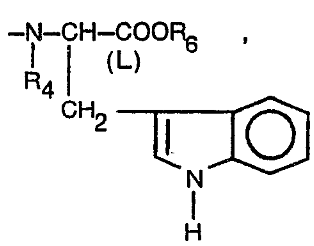 , 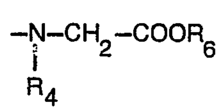 , 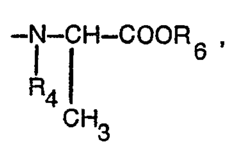 ,

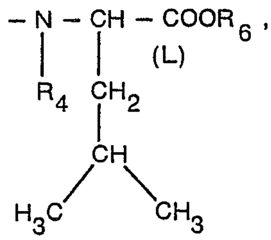, 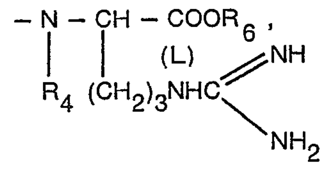

or 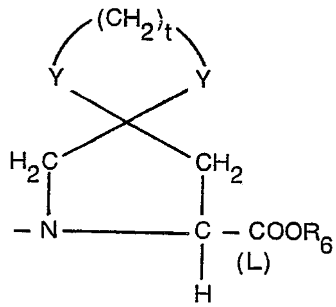 .

$R_4$ is hydrogen, methyl, cyclohexyl, phenyl or benzyl.
$R_6$ is hydrogen,

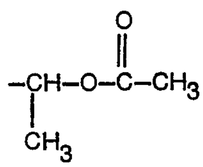 , 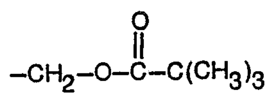 , 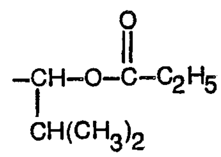 ,

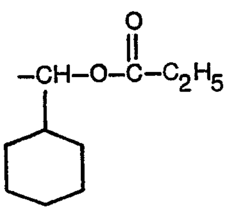 , 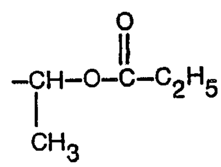 ,

or an alkali metal.

Y is oxygen or sulfur and t is two or three, especially wherein Y is sulfur and t is two.

Preferred compounds of this invention with respect to the phosphonamidate alkanoyl sidechain of the structure of formula I are those wherein:

$R_1$ is hydrogen or alkyl of 1 to 4 carbons, especially hydrogen or methyl.

$R_2$ is hydrogen, alkyl of 1 to 4 carbons, $CF_3$, or $(CH_2)_r$—$NH_2$ wherein R is 1 to 4, especially hydrogen, methyl or —$(CH_2)_4NH_2$, or $R_1$ and $R_2$ taken together are —$(CH_2)_3$—.

$R_3$ is hydrogen, an alkali metal, lower alkyl of 1 to 4 carbons, or

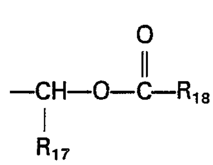

wherein $R_{17}$ is hydrogen, straight or branched chain lower alkyl of 1 to 4 carbons, or cyclohexyl and $R_{18}$ is straight or branched chain lower alkyl of 1 to 4 carbons or phenyl; especially hydrogen, an alkali metal, ethyl,

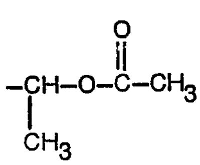 , 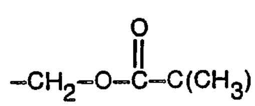 , 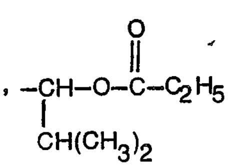 ,

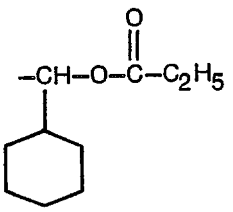, or 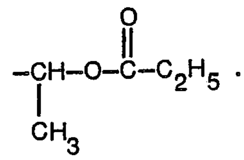.

$R_{21}$ is alkyl of 1 to 10 carbons;

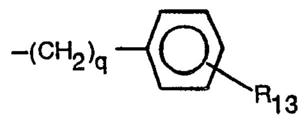

wherein q is zero or an integer from 1 to 4 and $R_{13}$ is hydrogen, methyl, methoxy, methylthio, chloro, bromo, fluoro, or hydroxy; —$(CH_2)_q$-cycloalkyl wherein cycloalkyl is of 5 or 6 carbons and q is zero or an integer from 1 to 4;

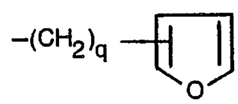

wherein q is zero or an integer from 1 to 4;

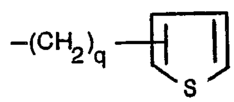

wherein q is zero or an integer from 1 to 4;

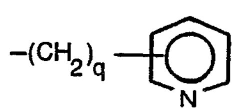

wherein q is zero or an integer from 1 to 4; or —$(CH_2)_s$—$NH_2$ wherein s is an integer from 1 to 8.

The compounds of this invention wherein at least one of $R_3$ or $R_6$ is hydrogen, form basic salts with various inorganic and organic bases which are also within the scope of the invention. Such salts include ammonium salts, alkali metal salts like lithium, sodium and potassium salts (which are preferred), alkaline earth metal salts like calcium and magnesium salts, salts with organic bases, e.g., dicyclohexylamine salt, benzathine, N-methyl-D-glucamine, hydrabamine salts, salts with amino acids like arginine and lysine. The nontoxic, physiologically acceptable salts are preferred, although other salts are also useful, e.g., in isolating or purifying the product. The salts are formed using conventional techniques.

As shown above, the amino or imino acid or ester portion of the molecule of the products of formula I represented by X is in the L-configuration. Depending upon the definition of $R_2$ and $R_{17}$ other asymmetric center may be present in the phosphonamidate alkanoyl sidechain. Thus, some of the compounds can accordingly exist in diastereoisomeric forms or in mixtures thereof. The above described processes can utilize racemates, enantiomers or diastereomers as starting materials. When diastereomeric products are prepared, they can be separated by conventional chromatographic or fractional crystallization methods.

The compounds of formula I, and the physiologically acceptable salts thereof, are hypotensive agents. They inhibit the conversion of the decapeptide angiotensin I to angiotensin II and, therefore, are useful in reducing or relieving angiotensin related hypertension. The action of the enzyme renin on angiotensinogen, a pseudoglobulin in blood pressure, produces angiotensin I. Angiotensin I is converted by angiotensin converting enzyme (ACE) to angiotensin II. The latter is an active pressor substance which has been implicated as the causative agent in several forms of hypertension in various mammalian species, e.g., humans. The compounds of this invention intervene in the angiotensinogen → (renin) → angiotensin

I→ angiotensin II sequence by inhibiting angiotensin converting enzyme and reducing or eliminating the formation of the pressor substance angiotensin II. Thus by the administration of a composition containing one (or a combination) of the compounds of this invention, angiotensin dependent hypertension in a species of mammal (e.g., humans) suffering therefrom is alleviated. A single dose, or preferably two to four divided daily doses, provided on a basis of about 0.1 to 100 mg. per kilogram of body weight per day is appropriate to reduce blood pressure. The substance is preferably administered orally, but parenteral routes such as the subcutaneous, intramuscular, intravenous or intraperitoneal routes can also be employed.

The compounds of this invention can also be formulated in combination with a diuretic for the treatment of hypertension. A combination product comprising a compound of this invention and a diuretic can be administered in an effective amount which comprises a total daily dosage of about 30 to 600 mg, preferably about 30 to 330 mg of a compound of this invention, and about 15 to 300 mg, preferably about 15 to 200 mg of the diuretic, to a mammalian species in need thereof. Exemplary of the diuretics contemplated for use in combination with a compound of this invention are the thiazide diuretics, e.g., chlorothiazide, hydrochlorothiazide, flumethiazide, hydroflumethiazide, bendroflumethiazide, methyclothiazide, trichloromethiazide, polythiazide or benzthiazide as well as ethacrynic acid, ticrynafen, chlorthalidone, furosemide, musolimine, bumetanide, triamterene, amiloride and spironolactone and salts of such compounds.

The compounds of formula I can be formulated for use in the reduction of blood pressure in compositions such as tablets, capsules or elixirs for oral administration, or in sterile solutions or suspensions for parenteral administration. About 10 to 500 mg of a compound of formula I is compounded with physiologically acceptable vehicle, carrier, excipient, binder, preservative, stabilizer, flavor, in a unit dosage form as called for by accepted pharmaceutical practice. The amount of active substance in these compositions or preparations is such that a suitable dosage in the range indicated is obtained.

The following examples are illustrated of the invention. Temperatures are given in degrees centigrade. AG-50W-X8 refers to a crosslinked polystyrene-divinylbenzene sulfonic acid cation exchange resin. HP-20 refers to a porous crosslinked polystyrene-divinyl benzene polymer resin.

Example 1

(S)-7-[N-[Hydroxy(4-phenylbutyl)phosphinyl]-L-alanyl]-1,4-dithia-7-azospiro[4.4]nonane-8-carboxylic acid, dilithium salt

a) (S)-1,4-Dithia-7-azaspiro[4.4]nonane-8-carboxylic acid, methyl ester, hydrochloride

Methanol (100 ml) is cooled in an ice bath and treated with acetyl chloride (5 ml) followed by 4,4-[ethylenebis(thio)]-L-propline, hydrochloride salt (9.5 g, 39 mmole). The mixture is stirred at room temperature overnight. The dark brown solution is treated with activated charcoal (8 g), filtered through Celite (Registered Trade Mark) and evaporated to give after trituration with ethyl acetate, a yellow-brown solid. This is recrystallized from methanol-ether (2:3) to give 6.65 g of (S)-1,4-dithia-7-azaspiro[4.4]nonane-8-carboxylic acid, methyl ester, hydrochloride as a pale yellow solid, m .p. 158—161°. Tlc (dichloromethane/methanol/acetic acid; 8:1:1) shows a single spot at $R_f$ 0.57.

b) (S)-7-[N-[(1,1-Dimethylethoxy)carbonyl]-L-alanyl]-1,4-dithia-7-azaspiro[4.4]nonane-8-carboxylic acid, methyl ester

A solution of N-[(1,1-dimethylethoxy)carbonyl]-L-alanine (2.48 g, 13.1 mmol) in dry chloroform (25 ml) at −15° is treated with N-methylmorpholine (1.47 ml, 13.1 mmole) followed by isobutyl chloroformate (1.8 ml, 13.1 mmole). After 25 minutes, (S)-1,4-dithia-7-azaspiro[4.4]nonane-8-carboxylic acid, methyl ester, hydrochloride (3.35 g, 13.1 mmole) and N-methylmorpholine (1.47 ml, 13.1 mmole) are added; gas evolution is observed. The mixture is kept below −10° for one hour, then allowed to warm to room temperature, and stirred overnight under argon. The mixture is diluted with ethyl acetate, filtered, and the filtrate is evaporated to dryness. The residue is taken up in ethyl acetate, washed successively with 5% potassium bisulfate, saturated sodium bicarbonate, saturated sodium chloride, dried ($Na_2SO_4$), filtered, and evaporated. The residue (5.6 g) is purified by flash chromatography on silica gel (200 g) eluting with ethyl acetate-hexane (1:2) to give 4.5 g of (S)-7-[N-[(1,1-dimethylethoxy)carbonyl]-L-alanyl]-1,4-dithia-7-azaspiro[4.4]nonane-8-carboxylic acid, methyl ester as a colorless foam. Tlc (ethyl acetate/hexane; 1:1) shows a single spot at $R_f$ 0.28.

c) (S)-7-(L-Alanyl)-1,4-dithia-7-azospiro[4.4]nonane-8-carboxylic acid, methyl ester, hydrochloride salt

A solution of the product from part (b) (4.5 g, 11.5 mmole) in distilled trifluoroacetic acid (15 ml) is stirred at room temperature under argon for 30 minutes. Excess trifluoroacetic acid is removed *in vacuo* [0.2 mbar (0.15 mm of Hg.)] for 2 hours to give 4.6 g. of colorless oil. This oil is dissolved in ethyl acetate (5 ml) and treated with a saturated solution of dry hydrochloric acid in ethyl acetate (30 ml). The resulting precipitate is collected and washed with ethyl acetate to give 3.2 g of (S)-7-(L-alanyl)-1,4-dithia-7-azaspiro[4.4]nonane-8-carboxylic acid, methyl ester, hydrochloride salt as a colorless crystalline solid, m.p. 205—206°. Tlc (dichloromethane/methanol/acetic acid; 8:1:1) shows a single spot at $R_f$ 0.44.

d) (S)-7-[N-[Ethoxy(4-phenylbutyl)phosphinyl]-L-alanyl]-1,4-dithia-7-azaspiro[4.4]nonane-8-carboxylic acid, methyl ester

A half saturated solution of dry chlorine in carbon tetrachloride is added dropwise by syringe to a solution of (4-phenylbutyl)phosphinic acid, phenylmethyl ester (1.9 g, 8.4 mmole), from Example 1(c), in 6 ml. of dry carbon tetrachloride under argon until the yellow color of excess chlorine persists. The mixture is evaporated to dryness [0.2 mbar (0.15 mm of Hg.)], and the colorless residue is taken up in 12 ml of dry tetrahydrofuran and treated with (S)-7-(L-alanyl)-1,4-dithia-7-azaspiro[4.4]nonane-8-carboxylic acid, methyl ester, hydrochloride salt (2.74 g, 8.4 mmole). The resulting suspension is cooled to 0° (ice bath) under argon and treated dropwise with a solution of triethylamine (2.7 ml) in 12 ml. of dry tetrahydrofuran over a 15 minute period. After the addition is complete, the mixture is stirred at 0° for 15 minutes, then allowed to warm to room temperature and stirred for an additional 45 minutes. The mixture is diluted with ethyl acetate, filtered and evaporated to dryness. The residue is taken up in ethyl acetate, washed successively with 5% potassium bisulfate, saturated sodium bicarbonate, saturated sodium chloride, dried ($Na_2SO_4$), and evaporated. The residue (4 g) is purified by flash chromatography on silica gel (140 g) eluting with hexane/acetone (5:3) to give 2.7 g. of (S)-7-[N-[ethoxy(4-phenylbutyl)phosphinyl]-L-alanyl]-1,4-dithia-7-azaspiro[4.4]nonane-8-carboxylic acid, methyl ester as a colorless oil. Tlc (dichloromethane/acetone; 3:2) shows a single spot at $R_f$ 0.48.

e) (S)-7-[N-[Ethoxy(4-phenybutyl)phosphinyl]-L-alanyl]-1,4-dithia-7-azaspiro[4.4]nonane-8-carboxylic acid

A solution of the methyl ester product from part (d) (1.5 g, 2.9 mmole) in dioxane (4 ml) is treated with 9 ml. of lithium hydroxide solution (0.96 N) and stirred in an atmosphere of argon at room temperature for 30 minutes. The reaction mixture is diluted with water and washed with ethyl acetate. The aqueous layer is acidified to pH 4 by the addition of 10% citric acid and the separated oil is extracted with ethyl acetate (2 × 150 ml). The organic layer is washed with water, brine, dried ($Na_2SO_4$) filtered and the solvent removed to give 1.46 g of (S)-7-[N-[ethoxy(4-phenylbutyl)phosphinyl]-L-alanyl]-1,4-dithia-7-azaspiro[4.4]nonane-8-carboxylic acid. Tlc (dichloromethane/methanol; 5:1) shows a single spot at $R_f$ 0.32.

f) (S)-7-[N-[Hydroxy(4-phenylbutyl)phosphinyl]-L-alanyl]-1,4-dithia-7-azaspiro[4.4]nonane-8-carboxylic acid, dilithium salt

A solution of the monoacid product from part (e) (1.4 g, 2.8 mmole) in dry dichloromethane (10 ml) is treated with bis(trimethylsilyl)acetamide (1 ml, 4 mmole) and stirred at room temperature under argon for 1.5 hours. The mixture is evaporated to dryness [0.2 mbar (0.15 mm of Hg.)], taken up in dry dichloromethane (15 ml), treated with bromotrimethylsilane (0.9 ml, 6.8 mmole) and stirred overnight at room temperature under argon. The mixture is again evaporated to dryness [0.2 mbar (0.15 mm of Hg.)] and the residue treated with a mixture of methanol (8 ml)—water (2 ml)—triethylamine (4 ml) and stirred at room temperature under argon for 30 minutes. The mixture is evaporated to dryness, taken up in water and passed through an AG—50W—X8($Li^+$) column (50 ml) eluting with water. Fractions containing the dilithium salt product are combined and lyophilized to give 1.4 g of crude white solid. This material is purified on an HP—20 column (200 ml bed volume) eluting with a linear gradient of water (100%)—acetonitrile (100%) at a flow rate of 5 ml/min collecting 5 ml fractions. The fractions containing the desired product are combined, filtered, and lyophilized to give 1 g of (S)-7-[N-[hydroxy(4-phenylbutyl)phosphinyl]-L-alanyl]-1,4-dithia-7-azaspiro[4.4]nonane-8-carboxylic acid, dilithium salt as a white free flowing powder. Tlc (isopropanol/conc. $NH_4OH$/water; 7:2:1) shows a single spot at $R_f$ 0.41.

Anal. calc'd for $C_{20}H_{27}O_5N_2S_2P \cdot 2Li \cdot 1.2\ H_2O$:

| | | | | |
|---|---|---|---|---|
| | C, 47.57; | H, 5.86; | N, 5.55; | P, 6.1 |
| Found: | C, 47.57; | H, 5.85; | N, 5.83; | P, 6.2. |

## Example 2

### N-[N-[Hydroxy(4-phenylbutyl)phosphinyl]-L-alanyl]-N-methylglycine, dilithium salt

a) N-Methylglycine, ethyl ester, hydrochloride

A suspension of N-methylglycine (10.0 g, 0.11 mole) in 120 ml of ethanol is saturated with dry hydrochloric acid gas at 0° (ice-bath), allowed to warm to room temperature, and stirred for 16 hours. Nitrogen is passed through the clear solution to discharge excess hydrochloric acid. The solution is evaporated to dryness and the residue is repeatedly triturated with dry ethyl ether. The resulting white solid is filtered off, washed with ethyl ether, and dried *in vacuo* over phosphorus pentoxide to give 16.7 g of N-methylglycine, ethyl ester, hydrochloride as a white solid; m.p. 119.5—121° (literature m.p. 122—123°).

b) N-[N-[(1,1-Dimethylethoxy)carbonyl]-L-alanyl]-N-methylglycine, ethyl ester

A mixture of N-[(1,1-dimethylethoxy)carbonyl]-L-alanine (2.48 g, 13.1 mmole) and dry chloroform (20 ml) at −20° under argon is treated with N-methylmorpholine (1.47 ml, 1.0 eq.) and isobutyl chloroformate (1.8 ml, 1.0 eq.) and stirred for 20 minutes. N-Methylglycine, ethyl ester, hydrochloride (2.0 g, 1.0 eq.) and N-methylmorpholine (1.47 ml) are added. After one hour, the ice-bath is removed and the reaction mixture is stirred for an additional 3 hours. The mixture is then partitioned between ethyl acetate/5% potassium bisulfate, and the organic phase is washed with 5% potassium bisulfate, saturated sodium bicarbonate, dried ($Na_2SO_4$), and evaporated to give 3.7 g of crude N-[N-(1,1-dimethylethoxy)carbonyl]-L-alanyl]-N-methylglycine, ethyl ester as an oil. Tlc (ethyl acetate/hexane, 1:2) shows a major spot at $R_f = 0.22$.

c) N-(L-Alanyl)-N-methylglycine, ethyl ester, hydrochloride

A mixture of the crude product from part (b) (3.7 g, 12.8 mmole), dichloromethane (10 ml.) and trifluoroacetic acid (10 ml) is stirred at 25° for 30 minutes. The dichloromethane and trifluoroacetic acid are evaporated and the resulting oil is taken up in ethyl ether (100 ml) and treated with saturated hydrochloric acid/ethyl ether in portions to precipitate the hydrochloride salt. The ethyl ether is decanted and the gummy residue is triturated with ethyl acetate/ethyl ether to give 2.4 g of N-(L-Alanyl)-N-methylglycine, ethyl ester, hydrochloride as a white crystalline solid; m.p. 150—151° (gas evolution). Tlc (acetic acid/methanol/dichloromethane; 1:1:8) shows a single spot at $R_f = 0.20$.

d) N-[N-[Ethoxy(4-phenylbutyl)phosphinyl]-L-alanyl]-N-methylglycine, ethyl ester

A mixture of (4-phenylbutyl)phosphonic acid, diethyl ester (0.97 g, 3.6 mmole), dry benzene (10 ml), and phosphorus pentachloride (0.75 g, 3.6 mmole) is refluxed under argon for 20 minutes. The phosphorus oxychloride and benzene are removed *in vacuo*, and the residue is taken up in dry tetrahydrofuran (10 ml) and treated with N-(L-alanyl)-N-methylglycine, ethyl ester, hydrochloride (0.67 g, 3.0 mmole). The resulting suspension is cooled to 0° (ice-bath) and treated dropwise with triethylamine (1.2 ml, 3.0 eq.) in tetrahydrofuran (5 ml) over a period of 2 minutes in an argon atmosphere. After stirring at 0° for 20 minutes the ice bath is removed and the reaction mixture is stirred for an additional 4 hours. The reaction mixture is then diluted with ethyl acetate and washed successively with 5% potassium bisulfate, saturated sodium bicarbonate, brine, dried ($MgSO_4$), and evaporated. The residue (1.4 g) is chromatographed on silica gel (60 g) eluting with toluene/acetone (1:1) to give 1.0 g of N-[N-[ethoxy(4-phenylbutyl)phosphinyl]-L-alanyl]-N-methylglycine, ethyl ester as an oil after evaporation. Tlc (toluene/acetone; 1:1) shows a single spot at $R_f = 0.19$.

e) N-[N-[Hydroxy(4-phenylbutyl)phosphinyl]-L-alanyl]-N-methylglycine, dilithium salt

A mixture of the diester product from part (d) (1.0 g, 2.4 mmole), trimethylsilylbromide (0.5 ml, 1.5 eq.), and dry dichloromethane (5 ml) is stirred under argon at room temperature for 16 hours. The dichloromethane and excess trimethylsilylbromide are evaporated *in vacuo*, the resulting oil is taken up in dry acetonitrile (10 ml), treated with 1N lithium hydroxide (6.0 ml, 2.5 eq.) and stirred at room temperature for 2 hours. The acetonitrile is evaporated, the solution is filtered and chromatographed on an HP—20 (200 ml) column eluting with a linear gradient water-acetonitrile (0 → 90% acetonitrile). The desired fractions are combined, evaporated to a small volume, filtered, and lyophilized to give 680 mg of N-[N-[hydroxy(4-phenylbutyl)phosphinyl]-L-alanyl]-N-methylglycine, dilithium salt as a white solid; darkens at 208°. Tlc (isopropanol/conc. $NH_4OH$/water; 7:2:1) shows a single spot at $R_f = 0.50$.

Anal. calc'd for $C_{16}H_{23}N_2O_5PLi_2 \cdot 2\ H_2O$

| | | | | |
|---|---|---|---|---|
| | C, 47.60; | H, 6.73; | N, 6.94; | P, 7.7 |
| Found: | C, 47.60; | H, 6.44; | N, 6.96; | P, 7.8. |

## Example 3

### N-[N-[Hydroxy(4-phenylbutyl)phosphinyl]-L-alanyl]-N-phenylglycine, dilithium salt

a) N-[N-[(1,1-Dimethylethoxy)carbonyl]-L-alanyl]-N-phenylglycine, ethyl ester

A solution of N-[(1,1-dimethylethoxy)carbonyl]-L-alanine (2.48 g, 13.1 mmole) in dry chloroform (20 ml) at −15° is treated with N-methyl morpholine (1.47 ml, 13.1 mmole) followed by isobutyl chloroformate (1.8 ml, 13.1 mmole). After 20 minutes, N-phenylglycine, ethyl ester (2.35 g, 13.1 mmole) is added. The mixture is kept below −10° for one hour, then allowed to warm to room temperature and stirred overnight under argon. The mixture is partitioned between ethyl acetate — 5% potassium bisulfate (75 ml each), and the organic phase is washed successively with 5% potassium bisulfate, saturated sodium bicarbonate, and saturated sodium chloride, dried ($Na_2SO_4$), and evaporated. The residue is purified by flash chromatography on silica gel (85 g) eluting first with dichloromethane and then ethyl acetate/dichloromethane (1:3) to give 1.72 g of N-[N-[(1,1-dimethylethoxy)carbonyl]-L-alanyl]-N-phenylglycine, ethyl ester as a colorless oil. Tlc (ethyl acetate-hexane; 1:2) shows a single spot at $R_f = 0.43$.

b) N-(L-Alanyl)-N-phenylglycine, ethyl ester, hydrochloride.

N-[N-[(1,1-Dimethylethoxy)carbonyl]-L-alanyl]-N-phenylglycine, ethyl ester (1.7 g, 4.86 mmole) is treated with a saturated solution of hydrochloric acid in ethyl acetate (20 ml, saturated with dry hydrochloric acid at 0°) and stirred at 0° (ice-bath) for 50 minutes. A stream of nitrogen is then passed through the solution to remove excess hydrochloric acid and the solution is evaporated to dryness. The residue is taken up in ethyl acetate (approximately 15 ml), evaporated again and finally dried *in vacuo* to give 1.4 g of N-(L-alanyl)-N-phenylglycine, ethyl ester, hydrochloride as a white foam. Tlc (acetic acid-methanol-dichloromethane; 1:1:8), shows a major spot at $R_f = 0.5$, slight impurity (approximately 10%) at $R_f = 0.26$.

c) N-[N-[Ethoxy(4-phenylbutyl)phosphinyl]-L-alanyl]-N-phenylglycine, ethyl ester

A solution of (4-phenylbutyl)phosphonic acid, diethyl ester (1.08 g, 4.0 mmole) in dry benzene (8 ml) is

treated with phosphorus pentachloride (0.85 g, 4.09 mmole) and refluxed under argon for 50 minutes. The cooled solution is evaporated to dryness at room temperature [0.67 mbar (0.5 mm of Hg.)], taken up in dry benzene (approximately 5 ml) and evaporated again. The colorless residue is taken up in dry tetrahydrofuran (10 ml) and treated with the crude N-(L-alanyl)-N-phenylglycine, ethyl ester, hydrochloride (1.2 g, 4.19 mmole). The resulting mixture is cooled in an ice-bath and treated dropwise with a solution of triethylamine (1.7 ml, 12.3 mmole) in dry tetrahydrofuran (5 ml) over a period of 10 minutes. After stirring at 0° for 15 minutes and room temperature for one hour, the mixture is partitioned between ethyl acetate-5% potassium bisulfate. The organic phase is washed successively with saturated sodium bicarbonate, saturated sodium chloride, dried ($Na_2SO_4$), and evaporated. The residue (1.76 g) is purified by flash chromatography on silica gel eluting first with acetone-hexane (1:3) then acetone-hexane (2:3) to give 1.01 g of N-[N-[ethoxy(4-phenylbutyl)phosphinyl]-L-alanyl]-N-phenylglycine, ethyl ester as a colorless oil. Tlc (acetone-toluene; 1:1) shows a single spot at $R_f = 0.35$.

d) N-[N-[Hydroxy(4-phenylbutyl)phosphinyl]-L-alanyl]-N-phenylglycine, dilithium salt

A solution of the diethyl ester product from part (c) (1.01 g, 2.13 mmole) in dry dichloromethane (3.0 ml.) is treated with trimethylsilylbromide (0.65 g, 4.93 mmole) and stirred at room temperature under argon for 16 hours. The mixture is then evaporated to dryness [0.67 mbar (0.5 mm Hg.)] and the residue treated with 1N lithium hydroxide (6.5 ml, 6.5 mmole) and dioxane (4 ml) and stirred at room temperature for 1.5 hours. The mixture is concentrated to a small volume taken up in water and filtered. The filtrate is chromatographed on an HP—20 column (200 ml, bed volume) eluting with a linear gradient of water-acetonitrile (0 → 100% acetonitrile) at a flow rate of 5 ml/min collecting 5 ml fractions. Fractions containing the desired product are pooled, evaporated, taken up in water, filtered and lyophilized to give 0.60 g of N-[N-[hydroxy(4-phenylbutyl)phosphinyl]-L-alanyl]-N-phenylglycine, dilithium salt as a fluffy white solid; m.p.: shrinks at 200°, darkens at 220°, m.p. greater than 250°. Tlc (isopropanol—conc. $NH_4OH$—water; 7:2:1) shows a single spot at $R_f = 0.51$.

Anal. calc'd for: $C_{21}H_{25}N_2O_5PLi_2 \cdot 1.35\ H_2O$:

| | | | | |
|---|---|---|---|---|
| | C, 55.47; | H, 6.14; | N, 6.16; | P, 6.81 |
| Found: | C, 55.47; | H, 5.86; | H, 6.20; | P, 6.7. |

Example 4

N-[N-Hydroxy(4-phenylbutyl)phosphinyl]-L-alanyl]-N-(phenylmethyl)glycine, dilithium salt

a) N-[N-[(1,1-Dimethylethoxy)carbonyl]-L-alanyl]-N-(phenylmethyl)glycine, ethyl ester

A solution of N-[(1,1-dimethylethoxy)carbonyl]-L-alanine (2.48 13.1 mmole) in dry chloroform (20 ml) at −15° is treated with N-methyl morpholine, (1.47 ml, 13.1 mmole) followed by isobutyl chloroformate (1.8 ml, 13.1 mmole). After 20 minutes, N-(phenylmethyl)glycine, ethyl ester (2.53 g, 13.1 mmole) is added. The mixture is kept below −10° for one hour and at room temperature for 1.5 hours. The mixture is partitioned between ethyl acetate — 5% potassium bisulfate (75 ml each), and the organic phase is washed successively with 5% potassium bisulfate, saturated sodium bicarbonate, and saturated sodium chloride, dried ($Na_2SO_4$), and evaporated. The residue is purified by flash chromatography on silica gel (110 g) eluting first with ethyl acetate—hexane; (1:6) then ethyl acetate—hexane (1:3) to give 3.93 g of N-[N-[(1,1-dimethylethoxy)carbonyl]-L-alanyl]-N-(phenylmethyl)glycine, ethyl ester as a colorless oil. Tlc (ethyl acetate—hexane; 1:2) shows a single spot at $R_f = 0.40$.

b) N-(L-Alanyl)-N-(phenylmethyl)glycine, ethyl ester, hydrochloride

N-[N-[(1,1-Dimethylethoxy)carbonyl]-L-alanyl]-N-(phenylmethyl)glycine, ethyl ester (3.5 g, 9.6 mmole) is treated with a saturated solution of hydrochloric acid in ethyl acetate (30 ml, saturated with dry hydrochloric acid at 0°) and stirred at 0° (ice-bath) for 45 minutes. A stream of nitrogen is then passed through the solution to remove excess hydrochloric acid and the solution is evaporated to dryness. The solid residue is triturated with ethyl ether, collected and dried *in vacuo* to give 2.62 g of N-(L-alanyl)-N-(phenylmethyl)glycine, ethyl ester, hydrochloride as a white, slightly hygroscopic solid; m.p. 140—141°. An analytical sample is recrystallized from acetonitrile—ethyl ether; m.p. 141.5—142°. Tlc (acetic acid—methanol—dichloromethane; 1:1:8) shows a single spot at $R_f = 0.40$.

c) N-[N-[Ethoxy(4-phenylbutyl)phosphinyl]-L-alanyl]-N-(phenylmethyl)glycine, ethyl ester

A solution of (4-phenylbutyl)phosphonic acid, diethyl ester (1.08 g, 4.0 mmole) in dry benzene (8 ml) is treated with phosphorus pentachloride (0.85 g, 4.09 mmole) and refluxed under argon for 45 minutes. The cooled solution is evaporated to dryness at room temperature [0.67 mbar (0.5 mm of Hg)], taken up in dry benzene (approximately 5 ml) and evaporated again. The colorless residue is taken up in dry tetrahydrofuran (10 ml) and treated with N-(L-alanyl)-N-(phenylmethyl)glycine, ethyl ester, hydrochloride (1.2 g, 4.16 mmole). The resulting suspension is cooled in an ice-bath and treated dropwise with a solution of triethylamine (1.7 ml, 12.3 mmole) in dry tetrahydrofuran (5 ml) over a period of 10 minutes. After stirring at 0° for 15 minutes and room temperature for one hour, the mixture is partitioned between ethyl acetate-5% potassium bisulfate. The organic phase is washed successively with saturated sodium bicarbonate, saturated sodium chloride, dried ($Na_2SO_4$), and evaporated. The residue is purified by flash chromatography on silica (95 g.) eluting with acetone-hexane (1:2) to give 1.58 g of N-[N-[ethoxy(4-phenylbutyl)phosphinyl]-L-alanyl]-N-(phenylmethyl)glycine, ethyl ester as a colorless oil. Tlc (acetone—toluene; 1:1) shows a single spot at $R_f$ = 0.43.

d) N-[N-[Hydroxy(4-phenylbutyl)phosphinyl]-L-alanyl]-N-(phenylmethyl)glycine, dilithium salt

A solution of the diethyl ester product from part (c) (1.13 g, 2.32 mmole) in dry dichloromethane (3.0 ml) is treated with trimethylsilylbromide (0.70 ml, 5.3 mmole) and stirred at room temperature under argon for 16 hours. The mixture is then evaporated to dryness [0.67 mbar (0.5 mm Hg.)] and the residue treated with 1N lithium hydroxide (7.0 ml, 7.0 mmole) and acetonitrile (5.0 ml) and stirred at room temperature under argon for 1.5 hours. The mixture is concentrated to a small volume, diluted with water (approximately 5 ml) and filtered. The filtrate is chromatographed on an HP—20 column (200 ml bed volume) eluting with a linear gradient of water—acetonitrile (0 → 100% acetonitrile) at a flow rate of 5 ml/min collecting 5 ml fractions. Fractions containing the desired product are pooled, evaporated, taken up in water, filtered and lyophilized to give 0.82 g of N-[N-[hydroxy(4-phenylbutyl)phosphinyl]-L-alanyl]-N-(phenylmethyl)glycine, dilithium salt as a fluffy white solid; m.p.: gradually darkens above 208°. Tlc (isopropanol—conc. $NH_4OH$—water; 7:2:1) shows a single spot at $R_f$ = 0.58.

Anal. Calc'd. for $C_{22}H_{27}N_2O_5PLi_2 \cdot 0.5\ H_2O$:

| | | | | |
|---|---|---|---|---|
| | C, 58.29; | H, 6.23; | N, 6.18; | P, 6.83 |
| Found: | C, 58.23; | H, 6.24; | N, 6.28; | P, 6.8. |

## Example 5

### N-Cyclohexyl-N-[N-[hydroxy(4-phenylbutyl)phosphinyl]-L-alanyl]glycine, dilithium salt

a) N-(cyclohexyl)glycine, ethyl ester

A solution of ethyl bromoacetate (11.1 ml, 0.10 mole) in dry ether (40 ml) is added dropwise to a solution of cyclohexylamine (11.0 g, 0.11 mole) and triethylamine (17.0 ml, 0.12 mole) in dry ether (80 ml.) at 0° (ice-bath) under argon over a period of 30 minutes. The mixture is then allowed to warm to room temperature and stirred for 16 hours. The mixture is filtered and concentrated. The residue is taken up in dichloromethane, washed with saturated sodium bicarbonate and water, dried ($Na_2SO_4$) and evaporated. The resulting brown liquid (15.5 g) is purified by short path distillation to give 12.6 g of N-(cyclohexyl)glycine, ethyl ester as a colorless liquid; b.p. 70—75° (0.5 mm. of Hg). Tlc (10% methanol—dichloromethane) shows a single spot at $R_f$ = 0.61.

b) N-Cyclohexyl-N-[N-(1,1-dimethylethoxy)carbonyl]-L-alanyl]glycine, ethyl ester

A solution of N-[(1,1-dimethylethoxy)carbonyl]-L-alanine (2.48 g, 13.1 mmole) in dry chloroform (20 ml) at −15° is treated with N-methylmorpholine (1.47 ml, 13.1 mmole) followed by isobutyl chloroformate (1.8 ml, 13.1 mmole). After 20 minutes, N-(cyclohexyl)glycine, ethyl ester (2.45 g, 13.2 mmole) is added. The mixture is kept below −10° for one hour, then allowed to warm to room temperature and stirred overnight under argon. The mixture is partitioned between ethyl acetate—5% potassium bisulfate (75 ml each). The organic phase is washed successively with 5% potassium bisulfate, saturated sodium bicarbonate, and saturated sodium chloride, dried ($Na_2SO_4$), and evaporated. The residue (4.6 g) is purified by flash chromatography on silica gel (110 g) eluting with ethyl acetate—hexane (1:6) to give 3.70 g of N-cyclohexyl-N-[N-[(1,1-dimethylethoxy)carbonyl]-L-alanyl]glycine, ethyl ester as a colorless viscous oil. Tlc (ethyl acetate—hexane) shows a single spot at $R_f$ = 0.45.

c) N-(L-Alanyl)-N-(cyclohexyl)glycine, ethyl ester, hydrochloride

A solution of N-cyclohexyl-N-[N-(1,1-dimethylethoxy)carbonyl]-L-alanyl]glycine, ethyl ester (3.65 g, 10.3 mmole) in ethyl acetate (5 ml) at 0° (ice-bath) is treated with a saturated solution of hydrochloric acid in ethyl acetate (25 ml, saturated with dry hydrochloric acid at 0°) and stirred at 0° (ice-bath) for one hour. A stream of nitrogen is then passed through the solution to remove excess hydrochloric acid and the solution is evaporated to dryness. The residue is taken up in ethyl acetate (approximately 25 ml) evaporated again and finally dried *in vacuo* to give 3.0 g of N-(L-alanyl)-N-(cyclohexyl)glycine, ethyl ester, hydrochloride as a white foam. Tlc (acetic acid—methanol—dichlorimethane; 1:1:8) shows a single spot at $R_f$ = 0.48.

d) N-Cyclohexyl-N-[N-[ethoxy(4-phenylbutyl)phosphinyl]-L-alanyl]glycine, ethyl ester

A solution of (4-phenylbutyl)phosphonic acid, diethyl ester (1.08 g, 4.0 mmole) in dry benzene (8 ml) is treated with phosphorus pentachloride (0.85 g, 4.09 mmole) and refluxed under argon for 45 minutes. The cooled solution is evaporated to dryness at room temperature [0.67 mbar (0.5 mm of Hg)], taken up in dry benzene (approximately 5 ml) and evaporated again. The colorless residue is taken up in dry tetrahydrofuran (10 ml) and treated with N-(L-alanyl)-N-(cyclohexyl)glycine, ethyl ester, hydrochloride (1.22 g, 4.17 mmole). The resulting mixture is cooled in an ice-bath and treated dropwise with a solution of triethylamine (1.7 ml, 12.3 mmole) in dry tetrahydrofuran (5 ml) over a period of 10 minutes. After stirring at 0° for 15 minutes and room temperature for one hour, the mixture is partitioned between ethyl acetate—5% potassium bisulfate. The organic phase is washed successively with saturated sodium bicarbonate, saturated sodium chloride, dried ($Na_2SO_4$) and evaporated. The residue is purified by flash chromatography on silica gel (90 g) eluting with acetone—hexane (1:2) to give 1.82 g of N-cyclohexyl-N-[N-[ethoxy(4-phenylbutyl)phosphinyl]-L-alanyl]glycine, ethyl ester as a colorless oil. Tlc (acetone—hexane; 1:1) shows a single spot at $R_f = 0.32$.

e) N-Cyclohexyl-N-[N-[hydroxy(4-phenylbutyl)phosphinyl]-L-alanyl]glycine, dilithium salt

A solution of the diethyl ester product from part (d) (1.18 g, 2.46 mmole) in dry dichloromethane (3.0 ml) is treated with trimethylsilylbromide (0.75 ml, 5.69 mmole) and stirred at room tempertaure under argon for 16 hours. The mixture is then evaporated to dryness [0.67 mbar (0.5 mm Hg)] and the residue treated with 1N lithium hydroxide (10.1 ml, 10.0 mmole) and acetonitrile (8 ml) and stirred at room temperature for 3 hours. The mixture is concentrated to a small volume, taken up in water and filtered. The filtrate is chromatographed on an HP—20 column (200 ml bed volume) eluting with a linear gradient of water—acetonitrile (0 → 100% acetonitrile) at a flow rate of 5 ml/min collecting 5 ml fractions. Fractions containing the desired product are pooled, evaporated, taken up in water, filtered and lyophilized to give 0.78 g of N-cyclohexyl-N-[N-[hydroxy(4-phenylbutyl)phosphinyl]-L-alanyl]glycine, dilithium salt as a fluffy white solid; m.p.: darkens at 230°, m.p. > 280°. Tlc (isopropanol—conc. $NH_4OH$—water; 7:2:1) shows a single spot at $R_f = 0.58$.

Anal. calc'd. for $C_{21}H_{31}N_2O_5PLi_2 \cdot 1.6\ H_2O$:

| | | | | |
|---|---|---|---|---|
| | C, 54.22; | H, 7.41; | N, 6.02; | P, 6.66 |
| Found: | C, 54.22; | H, 7.17; | N, 5.94; | P, 6.7. |

Examples 6—31

Following the procedure of Example 1 to 5 but employing the phosphonochloridate shown in Col. I and the dipeptide ester shown in Col. II one obtains the diester product shown in Col. III. Both the $R_3$ and $R_6$ ester groups may be removed to yield the corresponding diacid or salt or in the case of Example 31 only the $R_3$ ester group may be removed.

Col. I

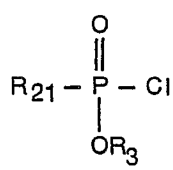

Col. II

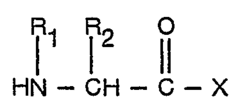

Col. III

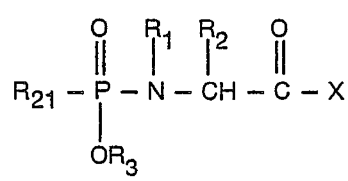

| Ex. | $R_{21}$ | $R_3$ | $R_1$ | $R_2$ | X |
|---|---|---|---|---|---|
| 6 | 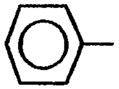 $-(CH_2)_4-$ | $-C_2H_5$ | $-CH_3$ | $-H$ | 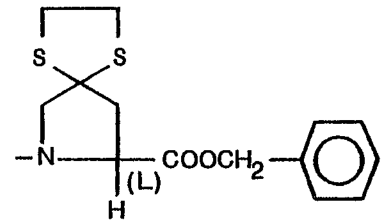 |
| 7 | 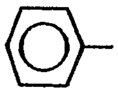 $-(CH_2)_2-$ | $-CH_2-$ 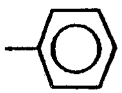 | $-H$ | $-CH_3$ | 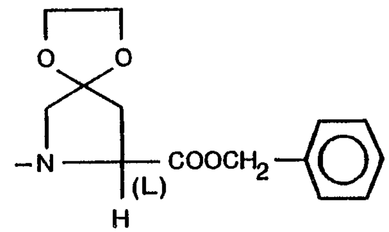 |
| 8 | 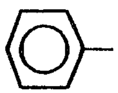 | $-CH_2-$ 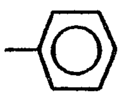 | $-H$ | $-CH_3$ | 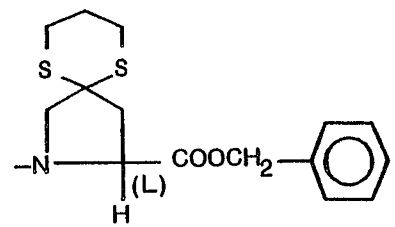 |
| 9 | $H_3C-(CH_2)_4-$ | $-CH_2-$ 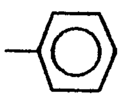 | $-H$ | $-CH_3$ | 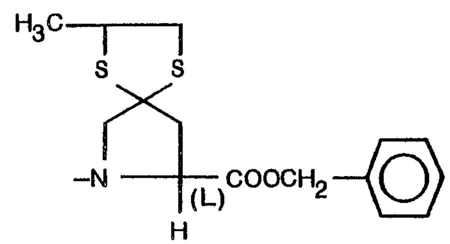 |

| Ex. | $R_{21}$ | $R_3$ | $R_1$ | $R_2$ | X |
|---|---|---|---|---|---|
| 10 | 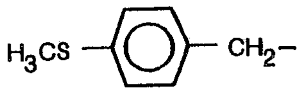 | 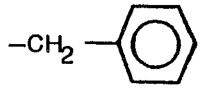 | −H | −H | 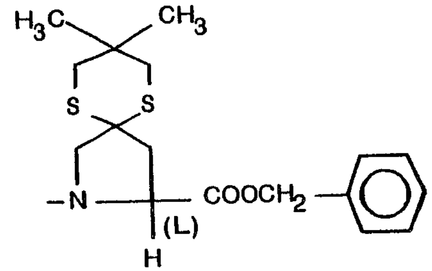 |
| 11 | 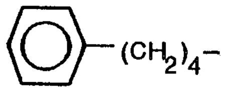 | 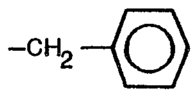 | −H | $-CH_3$ | 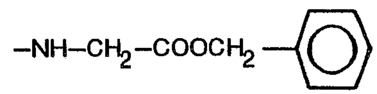 |
| 12 | 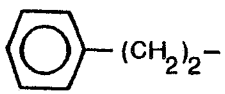 | 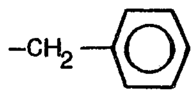 | −H | $-CH_3$ | 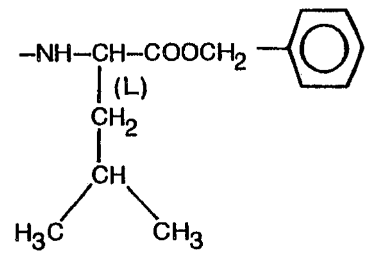 |
| 13 | 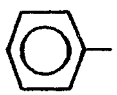 | 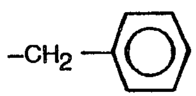 | −H | $-CH_3$ | 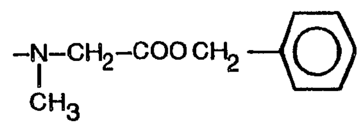 |
| 14 | 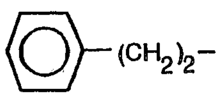 | $-C_2H_5$ | −H | $-CH_3$ | 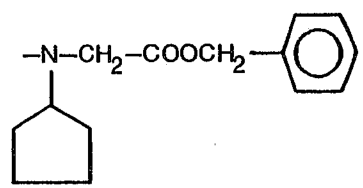 |

| Ex. | $R_{21}$ | $R_3$ | $R_1$ | $R_2$ | X |
|---|---|---|---|---|---|
| 15 | 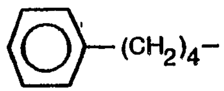 | 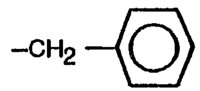 | $-H$ | $-CH_3$ | 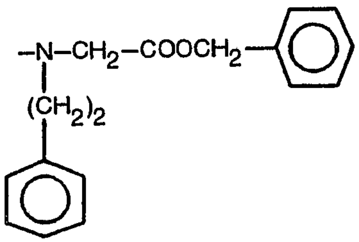 |
| 16 | $H_3C-(CH_2)_5-$ | $-C_2H_5$ | $-CH_3$ | $-H$ | 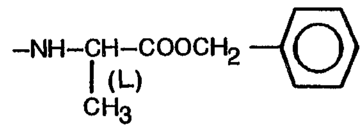 |
| 17 | 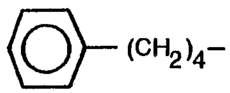 | $-C_2H_5$ | $-H$ | $-CH_3$ | 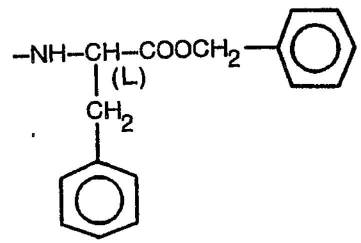 |
| 18 | 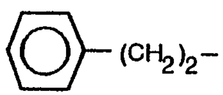 | 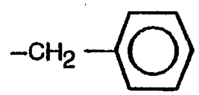 | $-H$ | $-CH_3$ | 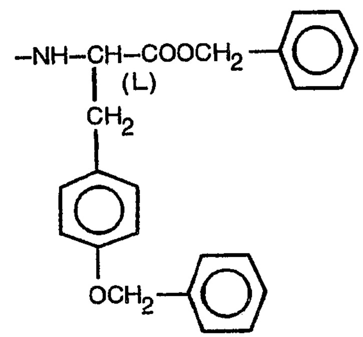 |

| Ex. | $R_{21}$ | $R_3$ | $R_1$ | $R_2$ | X |
|---|---|---|---|---|---|
| 19 | 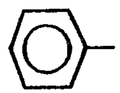 | $-C_2H_5$ | $-H$ | $-CH_3$ | 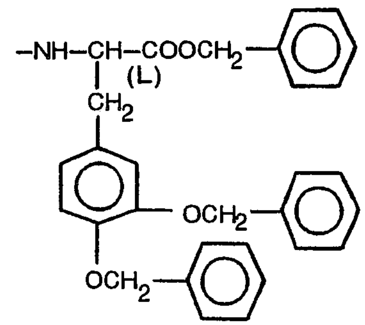 |
| 20 | $H_5C_2-$ | 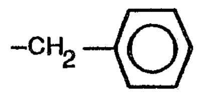 | $-H$ | $-CH_3$ | 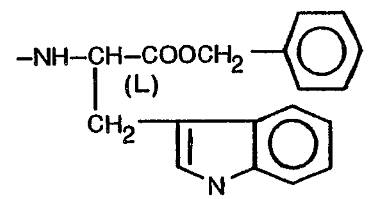 |
| 21 | 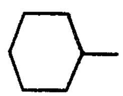 | 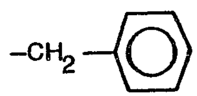 | $-H$ | $-CH_3$ | 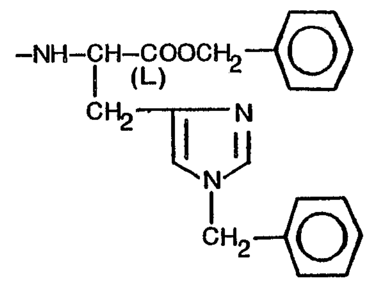 |
| 22 | 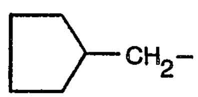 | 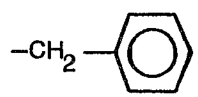 | $-H$ | $-CH_3$ | 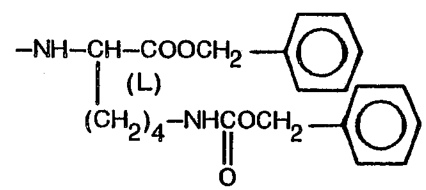 |

| Ex. | $R_{21}$ | $R_3$ | $R_1$ | $R_2$ | X |
|---|---|---|---|---|---|
| 23 | 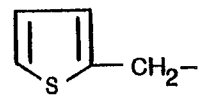 | $-C_2H_5$ | $-H$ | $-CH_3$ | 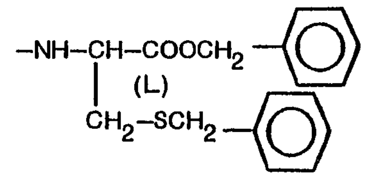 |
| 24 | 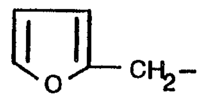 | 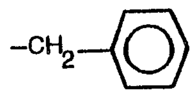 | $-H$ | $-CH_3$ | 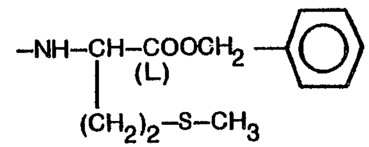 |
| 25 | 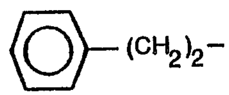 | 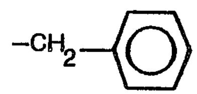 | $-H$ | $-CH_3$ | 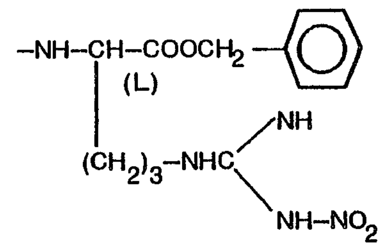 |
| 26 | 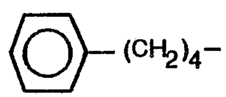 | $-C_2H_5$ | $-H$ | $-CH_3$ | 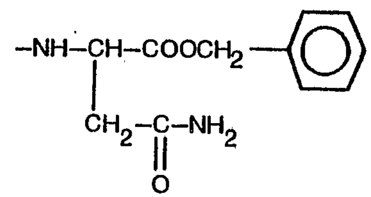 |
| 27 | $H_3C-(CH_2)_5-$ | 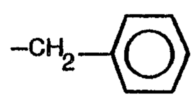 | $-H$ | $-CH_3$ | 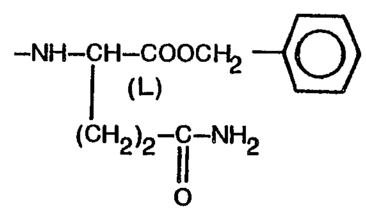 |

| Ex. | $R_{21}$ | $R_3$ | $R_1$ $R_2$ | X |
|---|---|---|---|---|
| 28 | 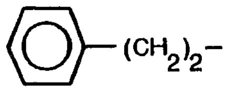 | 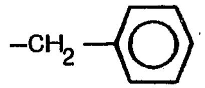 | $-(CH_2)_3-$ | 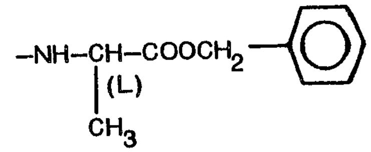 |
| 29 | $H_3C-(CH_2)_5-$ | 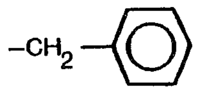 | $-(CH_2)_4$ | 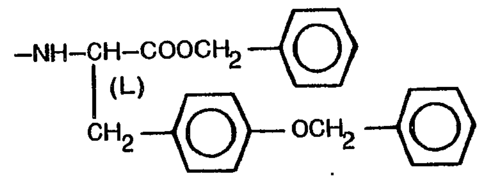 |
| 30 | $H_3C-CH_2-$ | 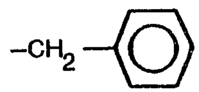 | $-(CH_2)_2-$ | 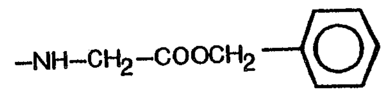 |
| 31 | 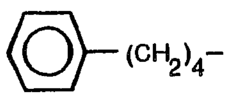 | 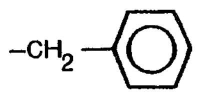 | $-(CH_2)_3-$ | 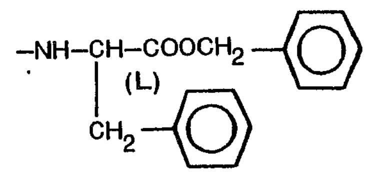 |

Examples 18, 19, 21—23, 25 and 29 are removed following completion of the coupling reaction.

Preparation 1

1-[N-[[(2,2-Dimethyl-1-oxopropoxy)methoxy]-(4-phenylbutyl)phosphinyl]-L-alanyl]-L-proline, lithium salt

a) 1-[N-[[(2,2-Dimethyl-1-oxopropoxy)methoxy]-(4-phenylbutyl)phosphinyl]-L-alanyl]-L-proline, phenylmethyl ester

A solution of 1-[N-[ethoxy(4-phenylbutyl)-phosphinyl]-L-alanyl]-L-proline, phenylmethyl ester (1.35 g, 2.7 mmole) in 10 ml of dry dichloromethane is treated with bromotrimethylsilane (0.9 ml) and stirred at room temperature under argon for 9.5 hours. The solution is evaporated to dryness (0.2 mm Hg) ($2.67 \cdot 10^{-4}$ bar) and the residue taken up in 5 ml of dioxane and treated with a solution of potassium bicarbonate (550 mg, 5.5 mmole) in 4 ml of water, stirred at room temperature for 10 minutes and evaporated to dryness. The residue is taken up in water and lyophilized. Tlc (isopropanol/conc. $NH_4OH$/water, 7:2:1) major spot at $R_f$ is 0.70.

The lyophilate is suspended in 10 ml of dry dimethylformamide treated with chloromethyl pivalate (0.8 ml, 5.5 mmole) and stirred at room temperature under argon. After 7 hours, additional chloromethyl pivalate (0.4 ml) and anhydrous potassium carbonate (0.3 g) are added and the resulting mixture is stirred overnight. The mixture is then diluted with ethyl acetate and washed successively with water, 5% potassium bisulfate, saturated sodium bicarbonate, and saturated sodium chloride solution, dried ($Na_2SO_4$), and evaporated. The residue is purified by flash chromatography on silica gel (100 g) eluting with acetone/hexane (3:8) to give 1-[N-[[(2,2-dimethyl-1-oxopropoxy)methoxy]-(4-phenylbutyl)phosphinyl]-L-alanyl]-L-proline, phenylmethyl ester as a colorless, viscous oil. Tlc (acetone/dichloromethane, 1:4) single spot at $R_f$ is 0.52.

b) 1-[N-[[(2,2-Dimethyl-1-oxopropoxy)methoxy](4-phenylbutyl)phosphinyl]-L-alanyl]-L-proline, lithium salt

A solution of the phenylmethyl ester from part (a) (0.52 g, 0.89 mmole) in 30 ml of ethyl acetate is treated with 10% palladium on carbon catalyst (300 mg) and hydrogenated in a Parr apparatus at an initial pressure of 48 psi. (3.36 bar) for 2.5 hours. The mixture is filtered through Celite (Registered Trade Mark) and evaporated to dryness. The residue is taken up in 5 ml of dichloromethane treated with triethylamine (0.15 ml, 1.08 mmole) and evaporated to dryness. The resulting triethylammonium salt is taken up in water, applied to an AG—50W—X8 ($Li^+$) column (30 ml bed volume) and eluted with water. The fractions containing the product are combined, millipore filtered and lyophilized to give 1-[N-[[(2,2-dimethyl-1-oxopropoxy)methoxy](4-phenylbutyl)phosphinyl]-L-alanyl]-L-proline, lithium salt as a white solid. Tlc (10% methanol/dichloromethane) single spot at $R_f$ is 0.48. 1R (KBr): 1750, 1630 $cm^{-1}$ (C=O).

Anal. Calc'd. for $C_{24}H_{36}N_2O_7PLi \cdot 0.8\ H_2O$:

| | | | | |
|---|---|---|---|---|
| | C, 55.77; | H, 7.33; | N, 5.42; | P, 5.99 |
| Found: | C, 55.77; | H, 7.39; | N, 5.43; | P, 5.82. |

Preparation 2

1-[N-[[(2,2-Dimethyl-1-oxopropoxy)methoxy]hexylphosphinyl]-L-alanyl]-L-proline, monolithium salt

a) 1-[N-[Ethoxy(hexylphosphinyl)]-L-alanyl]-L-proline, phenylmethyl ester

A solution of hexylphosphonic acid, diethyl ester (2.22 g, 10 mmole) in dry benzene (15 ml) is treated with phosphorus pentachloride (2.2 g, 11 mmole) and refluxed under argon for one hour. The solvent is removed under reduced pressure. Toluene (5 ml) is added and evaporated to remove traces of phosphorus oxychloride. The residue is dissolved in dichloromethane (20 ml), treated with L-alanyl-L-proline, phenylmethyl ester, hydrochloride (3.12 g, 10 mmole), cooled in ice and treated dropwise with a solution of triethylamine (3.75 ml) in dichloromethane (5 ml) during 10 minutes. The reaction mixture turns slightly rosy after the addition is completed. The reaction mixture is stirred at room temperature for 40 minutes, diluted with ethyl acetate, filtered and the solvent removed. The residue is once again taken up in ethyl acetate (120 ml), washed successively with water, 10% potassium bisulfate, saturated sodium bicarbonate, water, brine, dried ($Na_2SO_4$), and evaporated. The residue (4.4 g) is purified by flash chromatography on silica gel (135 g) eluting with hexane—acetone (3:2) to give 3.0 g of 1-[N-[ethoxy(hexylphosphinyl)]-L-alanyl]-L-proline, phenylmethyl ester as a colorless oil. Tlc (ethyl acetate) shows a single spot at $R_f = 0.125$.

b) 1-[N-[[(2,2-Dimethyl-1-oxopropoxy)methoxy]hexylphosphinyl]-L-alanyl]-L-proline, phenylmethyl ester

A solution of the diester product of part (a) (2.7 g, 6 mmole) in dichloromethane (25 ml) is treated with trimethylsilylbromide (2 ml) and stirred at room temperature in an atmosphere of argon. The volatile materials are removed under reduced pressure [0.2 mbar (0.15 mm of Hg)]. The residue is treated with a mixture of methanol (30 ml), water (5 ml) and triethylamine (5 ml) and stirred for 10 minutes. The solvents are removed under reduced pressure to give a triethylammonium salt (3.6 g) which is converted to the corresponding potassium salt by passing thorugh an AG—50W—X2($K^+$) column (200 ml bed) and eluting with water. The desired fractions are combined and lyophilized to give 2.63 g of 1-[N-[hydroxy(hexylphosphinyl)]-L-alanyl]-L-proline, phenylmethyl ester, monopotassium salt.

A suspension of 2.6 g of this potassium salt in dry dimethylformamide (35 ml) and potassium carbonate (1.5 g) is treated with chloromethylpivalate (1.7 g) and stirred overnight. Some additional chloromethylpivalate (1 g) and potassium carbonate (800 mg) are added and stirring continued for 36 hours. The

reaction mixture is diluted with ethyl acetate (200 ml), washed successively with water (6 × 50 ml), 10% potassium bisulfate, saturated sodium bicarbonate, and brine, dried ($Na_2SO_4$), and evaporated. The residue (5 g) is purified by flash chromatography on silica gel (140 g) eluting with ethyl acetate to give 1.9 g of 1-[N-[[(2,2-dimethyl-1-oxopropoxy)methoxy]hexylphosphinyl]-L-alanyl]-L-proline, phenylmethyl ester as a colorless oil. Tlc (dichloromethane-acetone; 4:1) shows a single spot at $R_f$ = 0.28.

c) 1-[N-[[(2,2-Dimethyl-1-oxopropoxy)methoxy]hexylphosphinyl]-L-alanyl]-L-proline, monolithium salt

A solution of the diester product from part (b) (1.19 g, 2 mmole) in ethyl acetate (45 ml) is treated with 10% palladium on carbon catalyst (500 mg) and hydrogenated in a Parr apparatus at 3.1 bar (45 psi) for 3 hours at room temperature. The reaction mixture is filtered (Celite), the bed is washed thoroughly with ethyl acetate and the solvent removed to give 920 mg of 1-[N-[[(2,2-dimethyl-1-oxopropoxy)-methoxy]hexylphosphinyl]-L-alanyl]-L-proline which is dissolved in dichloromethane (5 ml) containing triethylamine (300 mg). The solvent is removed and the resulting triethylammonium salt is passed through a column of AG—50W—X8($Li^+$) (50 ml settled volume) resin eluting with water. The fractions containing the desired product are combined, filtered and lyophilized to give 500 mg of 1-[N-[[(2,2-dimethyl-1-oxopropoxy)methoxy]hexylphosphinyl]-L-alanyl]-L-proline, monolithium salt as a colorless solid. Tlc (dichloromethane-methanol-acetic acid; 20:1:1) shows a single spot at $R_f$ = 0.42.

Anal. calc'd. for $C_{20}H_{36}N_2O_7P \cdot Li$

| | | | | |
|---|---|---|---|---|
| | C, 52.84; | H, 7.99; | N, 6.16; | P, 6.81 |
| Found: | C, 52.60; | H, 8.25; | N, 6.10; | P, 6.6. |

Preparation 3

1-[N-[[(2,2-Dimethyl-1-oxopropoxy)methoxy]hexylphosphinyl]-L-lysyl]-L-proline, monolithium salt

a) 1-[$N^2$-[[(2,2-Dimethyl-1-oxopropoxy)methoxy]hexylphosphinyl]-$N^6$-[(phenylmethoxy)carbonyl]-L-lysyl]-L-proline, phenylmethyl ester

A solution of 1-[$N^2$-[ethoxy(hexyl)phosphinyl]-$N^6$-[(phenylmethoxy)carbonyl]-L-lysyl]-L-proline, phenylmethyl ester (3.5 g, 5.4 mmole) in dry dichloromethane (25 ml) is treated with trimethylsilylbromide (1.75 ml) and stirred at room temperature under argon overnight. Volatile materials are removed under vacuum (0.15 mm of Hg) ($2.10^{-4}$ bar), the residue (4.8 g) is taken up in a mixture of methanol (40 ml)-water (5 ml)-triethylamine (5 ml) and stirred at room temperature for 10 minutes. The solvents are removed in a rotary evaporator and the residue is dissolved in water (5 ml) and passed through an AG—50W-X2($K^+$) (75 ml settled volume) column and eluted with water. Fractions containing the desired product are combined, filtered and lyophilized to give 3.74 g of 1-[$N^2$-[hydroxy(hexyl)phosphinyl]-$N^6$-[(phenylmethoxy)carbonyl]-L-lysyl]-L-proline, phenylmethyl ester, monopotassium salt.

This crude potassium salt (3.74 g) is suspended in dry dimethylformamide (35 ml) containing anhydrous potassium carbonate (1.56 g) and treated with chloromethyl pivalate (1.7 ml, 11.3 mmole) and the mixture is stirred at room temperature overnight. Additional chloromethyl pivalate (0.85 ml) and anhydrous potassium carbonate (0.8 g) are added and the mixture is stirred at room temperature for 48 hours. The reaction mixture is diluted with ethyl acetate (250 ml), washed successively with water, 10% potassium bisulfate, saturated sodium bicarbonate, water, brine, dried ($Na_2SO_4$), filtered, and evaporated. The residue (3.6 g) is purified by flash chromatography on silica gel (120 g) eluting with ethyl acetate to give 1.7 g of 1-[$N^2$-[[(2,2-dimethyl-1-oxopropoxy)methoxy]hexylphosphinyl]-$N^6$-[(phenylmethoxy)carbonyl]-L-lysyl]-L-proline, phenylmethyl ester as a colorless oil.

b) 1-[N-[[(2,2-Dimethyl-1-oxopropoxy)methoxy]hexylphosphinyl]-L-lysyl]-L-proline, monolithium salt

A mixture of the diester product from part (a) (600 mg, 0.82 mmole), methanol (30 ml), water (3 ml), and 10% palladium on carbon catalyst (500 mg) is hydrogenated in a Parr apparatus at 3.45 bar (50 psi) for 2.5 hours. The catalyst is removed by filtration (Celite bed) and the solvent evaporated. The residue is taken up in ethyl acetate, treated with triethylamine (0.4 ml, 4.0 eq.) and the excess triethylamine and ethyl acetate are evaporated. The resulting triethylammonium salt is taken up in water and applied to an AG—50W—X8($Li^+$) (10 ml) column eluting with water. The fractions containing the desired product are combined, evaporated to a small volume and chromatographed on an HP—20 (200 ml) column eluting with a linear gradient of water—acetonitrile (0 → 90% acetonitrile). The fractions containing the desired product are combined, evaporated to dryness, taken up in water, filtered, and lyophilized to give 195 mg of 1-[N-[[(2,2-dimethyl-1-oxopropoxy)methoxy]hexylphosphinyl]-L-lysyl]-L-proline, monolithium salt as a white solid; m.p. 87—90°. Tlc (isopropanol—conc.$NH_4OH$—water; 7:2:1) shows a single spot at $R_f$ = 0.7.

Anal. calc'd for $C_{23}H_{43}N_3O_7PLi \cdot 0.84\ H_2O$:

| | | | | |
|---|---|---|---|---|
| | C, 52.46, | H, 8.55; | N, 7.98; | P, 5.9 |
| Found: | C, 52.46; | H, 8.68; | N, 7.86; | P, 6.0. |

Preparations 4—14

Following the procedure of Preparation 1 but substituting for the chloromethyl pivalate the alkylating agents listed below in Col. I, the products listed below in Col. II are obtained.

| Preparation | Col. I | Col. II |
|---|---|---|
| 4 | 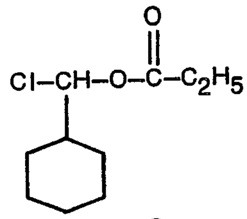 | 1-[N-[[Cyclohexyl(1-oxopropoxy)methoxy](4-phenylbutyl)phosphinyl]-L-alanyl]-L-proline, lithium salt |
| 5 | 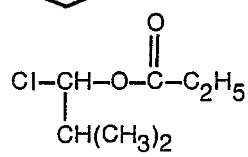 | 1-[N-[[(2-Methyl-1-(1-oxopropoxy)propoxy](4-phenylbutyl)phosphinyl]-L-alanyl]-L-proline, lithium salt |
| 6 | 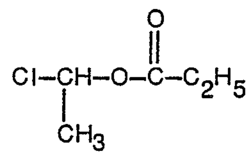 | 1-[N-[[1-(1-Oxopropoxy)ethoxy](4-(phenylbutyl)phosphinyl]-L-alanyl]-L-proline, lithium salt |
| 7 | 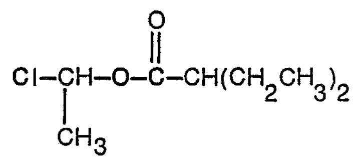 | 1-[N-[[1-(2-Ethyl-1-oxobutoxy)ethoxy](4-phenylbutyl)phosphinyl]-L-alanyl]-L-proline, lithium salt |
| 8 | 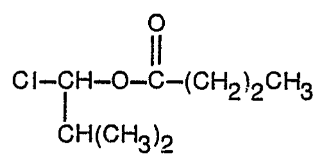 | 1-[N-[[2-Methyl-1-(1-oxobutoxy)propoxy](4-phenylbutyl)phosphinyl]-L-alanyl]-L-proline, lithium salt |
| 9 | 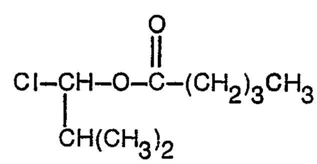 | 1-[N-[[2-Methyl-1-[(1-oxopentyl)oxy]-propoxy](4-phenylbutyl)phosphinyl]-L-alanyl]-L-proline, lithium salt |
| 10 | 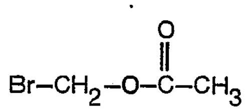 | 1-[N-[[(Acetyloxy)methoxy](4-phenylbutyl)phosphinyl]-L-alanyl]-L-proline, lithium salt |
| 11 | 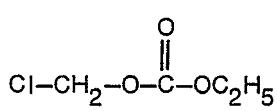 | 1-[N-[[(Ethoxycarbonyloxy)methoxy]-(4-phenylbutyl)phosphinyl]-L-alanyl]-L-proline, lithium salt |
| 12 | 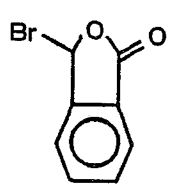 | 1-[N-[(1,3-Dihydro-3-oxo-1-isobenzofuranyloxy) (4-phenylbutyl)phosphinyl]-L-alanyl]-L-proline, lithium salt |

| Preparation | Col. I | Col. II |
|---|---|---|
| 13 | 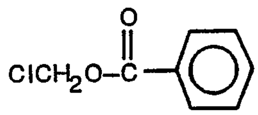 | 1-[N-[[(Phenylcarbonyloxy)methoxy]-(4-phenylbutyl)phosphinyl]-L-alanyl]-L-proline, lithium salt |
| 14 | 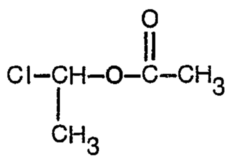 | 1-[N-[[1-(Acetyloxy)ethoxy](4-phenylbutyl)phosphinyl]-L-alanyl]-L-proline, lithium salt |

Similarly, the alkylating agents of Preparations 1 to 14 can be employed with ester products of Examples 1 to 30 to yield other compounds within the scope of this invention.

### Example 32

(S)-7-[N-[Hydroxy(4-phenylbutyl)phosphinyl]-L-alanyl]-1,4-dithia-7-azaspiro[4.4]nonane-8-carboxylic acid, disodium salt

Following the procedure of Example 1 but substituting AG—50W—X8 ($Na^+$) for the lithium resin in part (f), one obtains (S)-7-[N-[hydroxy(4-phenylbutyl)phosphinyl]-L-alanyl]-1,4-dithia-7-azaspiro[4.4]nonane-8-carboxylic acid, disodium salt.

This procedure can be employed in Examples 2—31 to give the corresponding mono or disodium salt. Similarly, by employing a potassium resin the corresponding mono or dipotassium salt is obtained.

### Example 33

1000 tablets each containing the following ingredients:

| | |
|---|---|
| (S)-7-[N-[hydroxy(4-phenylbutyl)phosphinyl]-L-alanyl]-1,4-dithia-7-azaspiro[4.4]-nonane-8-carboxylic acid | 100 mg |
| Corn starch | 50 mg |
| Gelatin | 7.5 mg |
| Avicel (Registered Trade Mark) (microcrystalline cellulose) | 25 mg |
| Magnesium stearate | 2.5 mg |
| | 185 mg |

are prepared from sufficient bulk quantities by mixing the (S)-7-[N-[hydroxy(4-phenylbutyl)phosphinyl]-L-alanyl]-1,4-dithia-7-azaspiro[4.4]nonane-8-carboxylic acid, disodium salt and corn starch with an aqueous solution of the gelatin. The mixture is dried and ground to a fine powder. The Avicel (Registered Trade Mark) and then the magnesium stearate are admixed with granulation. This mixture is then compressed in a tablet to form 1000 tablets each containing 100 mg. of active ingredient.

In a similar manner, tablets containing 100 mg of the product of any of Examples 2 to 31 can be prepared.

Example 34

1000 tablets each containing the following ingredients:

| | |
|---|---|
| (S)-7-[N-[hydroxy(4-phenylbutyl)phosphinyl]-L-alanyl]-1,4-dithia-7-azaspiro[4.4]-nonane-8-carboxylic acid, disodium salt | 50 mg |
| Lactose | 25 mg |
| Avicel (Registered Trade Mark) | 38 mg |
| Corn starch | 15 mg |
| Magnesium stearate | 2 mg |
| | 130 mg |

are prepared from sufficient bulk quantites by mixing the (S)-7-[N-[hydroxy(4-phenylbutyl)phosphinyl]-L-alanyl]-1,4-dithia-7-azaspiro[4.4]nonane-8-carboxylic acid, disodium dalt, lactose and Avicel (Registered Trade Mark) and then blending with the corn starch. Magnesium stearate is added and the dry mixture is compressed in a tablet press to form 1000 tablets each containing 50 mg of active ingredient. The tablets are coated with a solution of Methocel (Registered Trade mark) E 15 (methyl cellulose) including as a color a lake containing yellow #6.

In a similar manner, tablets containing 50 mg of the product of any Examples 1 to 31 can be prepared.

Example 35

Two piece #1 gelatin capsules each containing 100 mg of (S)-7-[N-[hydroxy(4-phenylbutyl)-phosphinyl]-L-alanyl]-1,4-dithia-7-azaspiro[4.4]nonane-8-carboxylic acid, disodium salt are filled with a mixture of the following ingredients:

| | |
|---|---|
| (S)-7-[N-[hydroxy(4-phenylbutyl)phosphinyl]-L-alanyl]-1,4-dithia-7-azaspiro[4.4]-nonane-8-carboxylic acid, disodium salt | 100 mg |
| Magnesium stearate | 7 mg |
| Lactose | 193 mg |
| | 300 mg |

In a similar manner, capsules containing 100 mg of the product of any of Examples 1 to 31 can be prepared.

Example 36

An injectable solution is prepared as follows:

| | |
|---|---|
| (S)-7-[N-[hydroxy(4-phenylbutyl)phosphinyl]-L-alanyl]-1,4-dithia-7-azaspiro[4.4]-nonane-8-carboxylic acid, disodium salt | 500 g |
| Methyl paraben | 5 g |
| Propyl paraben | 1 g |
| Sodium chloride | 25 g |
| Water for injection | 5 l |

The active substance, preservatives, and sodium chloride are dissolved in 3 liters of water for injection

and then the volume is brought up to 5 liters. The solution is filtered through a sterile filter and asceptically filled into presterilized vials which are closed with presterilized rubber closures. Each vial contains 5 ml of solution in a concentration of 100 mg of active ingredient per ml of solution for injection.

In a similar manner, an injectable solution containing 100 mg of active ingredient per ml of solution can be prepared for the product of any Examples 1 to 31.

Example 37

1000 tablets each containing the following ingredients:

| | |
|---|---|
| (S)-7-[N-[hydroxy(4-phenylbutyl)phosphinyl]-L-alanyl]-1,4-dithia-7-azaspiro[4.4]-nonane-8-carboxylic acid, | 100 mg |
| Avicel (Registered Trade Mark) | 100 mg |
| Hydrochlorothiazide | 12.5 mg |
| Lactose | 113 mg |
| Corn starch | 17.5 mg |
| Stearic acid | 7 mg |
| | 350 mg |

are prepared from sufficient bulk quantites by slugging the (S)-7-[N-[hydroxy(4-phenylbutyl)phosphinyl]-L-alanyl]-1,4-dithia-7-azaspiro[4.4]nonane-8-carboxylic acid, disodium salt, Avicel (Registered Trade Mark) and a portion of the stearic acid. The slugs are ground and passed through a #2 screen, then mixed with the hydrochlorothiazide, lactose, corn starch, and remainder of the stearic acid. The mixture is compressed into 350 mg capsule shaped tablets in a tablet press. The tablets are scored for dividing in half.

In a similar manner, tablets can be prepared containing 100 mg of the product of any of Examples 1 to 31.

**Claims**

1. A compound of the formula

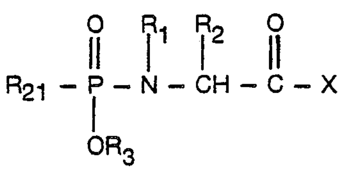

and pharmaceutically acceptable salts thereof wherein:

X is 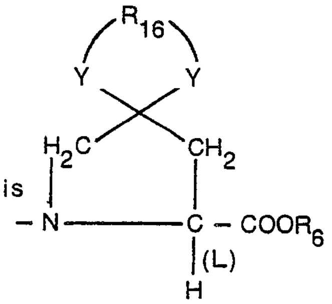 or 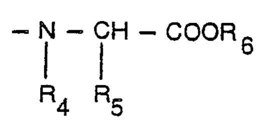 ;

Y is oxygen or sulfur;

$R_{16}$ is an ethylene or trimethylene radical in which one or more of the carbon atoms may have an alkyl of 1 to 4 carbon atoms of a di (alkyl of 1 to 4 carbon atoms) substitutent.

$R_4$ is hydrogen, lower alkyl, $-(CH_2)_m$-cycloalkyl, or

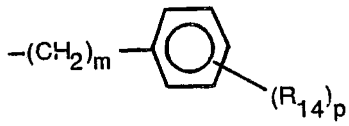

$R_5$ is hydrogen, lower alkyl,

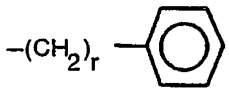 , 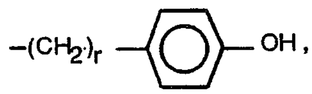

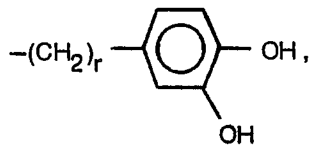 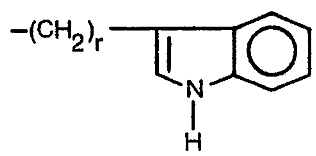 ,

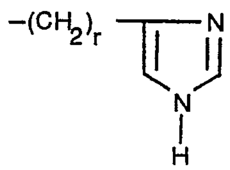 , $-(CH_2)_r-NH_2$ , $-(CH_2)_r-SH$ ,

$-(CH_2)_r-S-$lower alkyl , 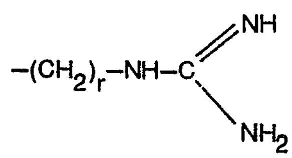 ,

or 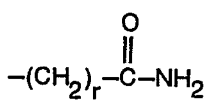 .

r is an integer from 1 to 4;
$R_1$ is hydrogen, lower alkyl, or cycloalkyl;
$R_2$ is hdyrogen, lower alkyl, halo substituted lower alkyl,

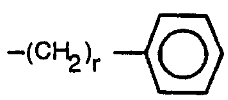 , 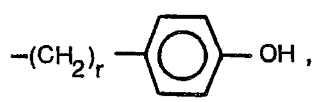 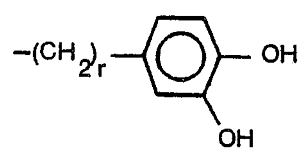

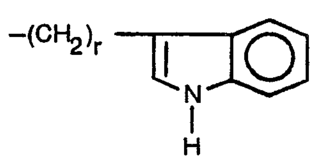 , 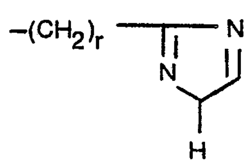 ,

$-(CH_2)_r-NH_2$ , $-(CH_2)_r-SH$ , $-(CH_2)_r-S-$lower alkyl ,

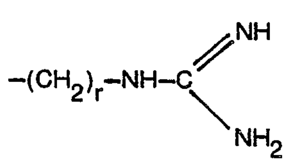 , 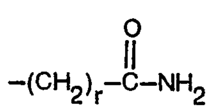 ,

or $R_1$ and $R_2$ taken together are $-(CH_2)_n-$ wherein n is an integer from 2 to 4;

$R_3$ and $R_6$ are independently selected from hydrogen, alkali metal, lower alkyl, benzyl, benzhydryl, or

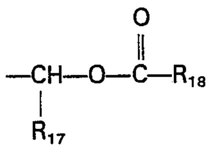

wherein $R_{17}$ is hydrogen, lower alkyl, cycloalkyl, or phenyl, and $R_{18}$ is hydrogen, lower alkyl, lower alkoxy, phenyl, or $R_{17}$ and $R_{18}$ taken together are $-(CH_2)_2-$, $-(CH_2)_3$, $-CH=CH-$, or

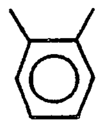 ;

$R_{21}$ is alkyl of 1 to 10 carbons,

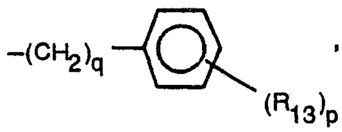 , $-(CH_2)_q-$cycloalkyl , $-(CH_2)_s-NH_2$ ,

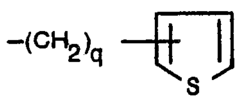 , 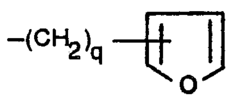 , or 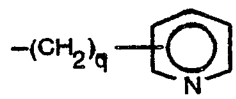

wherein q is zero or an integer from 1 to 7, s is an integer from 1 to 8;

$R_{13}$ is hydrogen, alkyl of 1 to 4 carbons, alkoxy of 1 to 4 carbons, alkylthio of 1 to 4 carbons, chloro, bromo, fluoro, trifluoromethyl, hydroxy, phenyl, phenoxy, phenylthio, or phenylmethyl;

$R_{14}$ is hydrogen, lower alkyl of 1 to 4 carbons, alkoxy of 1 to 4 carbons, alkylthio of 1 to 4 carbons, chloro, bromo, fluoro, trifluoromethyl, or hydroxy;

m is zero, one, two or three; and

p is one, two or three provided that p is more than one only if $R_{13}$ or $R_{14}$ is hydrogen, methyl, methoxy, chloro or fluoro.

2. A compound of Claim 1 wherein;

$R_4$ is hydrogen, methyl, cyclohexyl, phenyl or benzyl;

$R_5$ is hydrogen, alkyl of 1 to 4 carbons,

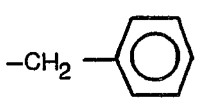 , 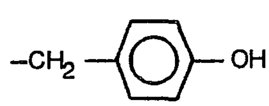 , 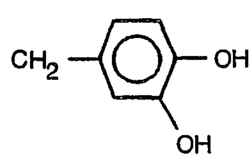 ,

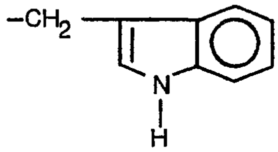 , 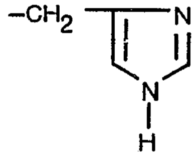 , $-(CH_2)_4-NH_2$ , $-CH_2SH$ ,

$-(CH_2)_2-S-CH_3$ , 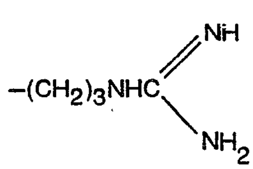 , 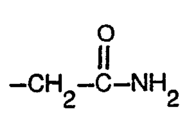 , or 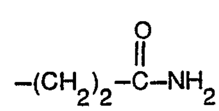 ;

Y is oxygen or sulfur;

$R_{16}$ is an ethylene or trimethylene radical in which one or more of the carbons has a methyl or dimethyl substituent;

m is zero, one or two;

$R_{13}$ is hydrogen, methyl, methoxy, methylthio, chloro, bromo, fluoro, or hydroxy;

$R_1$ is hydrogen or alkyl of 1 to 4 carbons;

$R_2$ is hydrogen, alkyl of 1 to 4 carbons, $CF_3$, or $(CH_2)_r—NH_2$, wherein r is 1 to 4, or $R_1$ and $R_2$ taken together are $—(CH_2)_3—$;

$R_{21}$ is alkyl of 1 to 10 carbons,

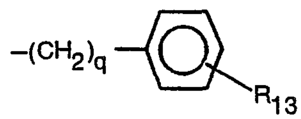

$—(CH_2)_q$-cycloalkyl wherein cycloalkyl is of 5 or 6 carbons,

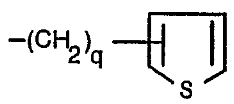 , 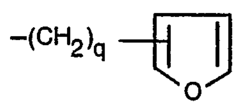 , 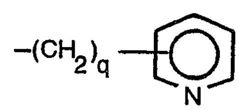 ,

or $—(CH_2)_s—NH_2$ wherein q is zero or an integer from 1 to 4, s is an integer from 1 to 8, and $R_{13}$ is as defined above;

$R_3$ and $R_6$ are independently selected from hydrogen, alkali metal, alkyl of 1 to 4 carbons, or

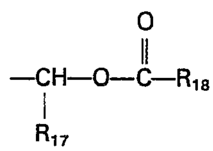

$R_{17}$ is hydrogen, straight or branched chain alkyl of 1 to 4 carbons, or cyclohexyl; and $R_{18}$ is straight or branched chain alkyl of 1 to 4 carbons or phenyl.

3. A compound of claim 1 or 2 wherein $R_{21}$ is alkyl of 1 to 10 carbons.

4. The compound of claim 3 wherein $R_{21}$ is methyl.

5. The compound of claim 3 wherein $R_{21}$ is $—(CH_2)_5—CH_3$.

6. The compound of claim 3 wherein $R_{21}$ is $—(CH_2)_7—CH_3$.

7. A compound of claim 1 or 2 wherein $R_{21}$ is

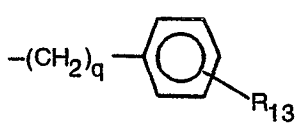

q is zero or an integer from 1 to 4, and $R_{13}$ is hydrogen, methyl, methoxy, methylthio, chloro, bromo, fluoro or hydroxy.

8. The compound of claim 7 wherein $R_{21}$ is phenyl.

9. The compound of claim 7 wherein $R_{21}$ is benzyl.

10. The compound of claim 7 wherein $R_{21}$ is phenethyl.

11. The compound of claim 7 wherein $R_{21}$ is 3-phenylpropyl.

12. The compound of claim 7 wherein $R_{21}$ is 4-phenylbutyl.

13. A compound of claim 1 or 2 wherein $R_{21}$ is $—(CH_2)_s—NH_2$ and s is an integer from 1 to 8.

14. The compound of claim $R_{13}$ wherein $R_{21}$ is $—(CH_2)_6—NH_2$.

15. A compound of any one of the preceding claims wherein $R_1$ is hydrogen.

16. A compound of any one of claims 1 to 14 wherein $R_1$ is methyl.

17. A compound of any one of the preceding claims wherein $R_2$ is hydrogen.

18. A compound of any one of claims 1 to 16 wherein $R_2$ is methyl.

19. A compound of any one of claims 1 to 16 wherein $R_2$ is $—(CH_2)_4—NH_2$.

20. A compound of any one of claims 1 to 14 wherein $R_1$ and $R_2$ taken together are $—(CH_2)_3—$.

21. A compound of any one of the preceding claims wherein X is

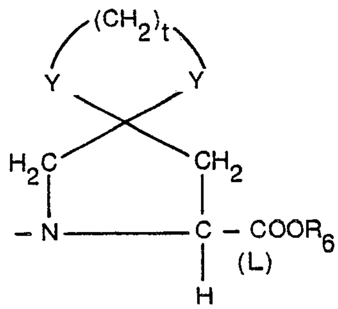

;

22. A compound of claim 21 wherein X is

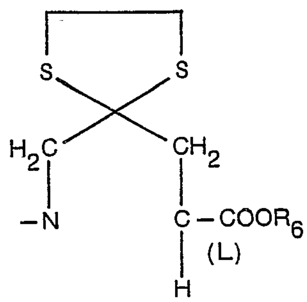

wherein $R_6$ is as defined in claims 1 or 2.

23. A compound of any one of claims 1 to 20 wherein X is

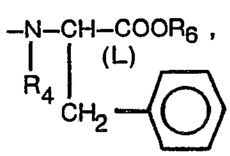
,
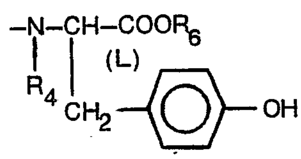

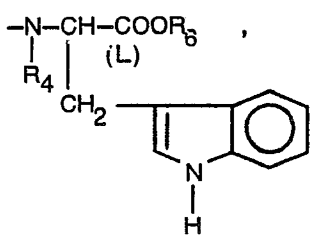
,
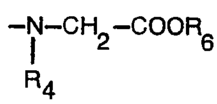
,

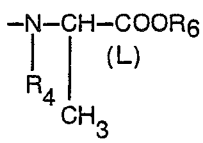
,
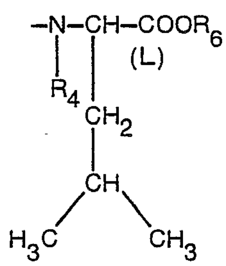
, or
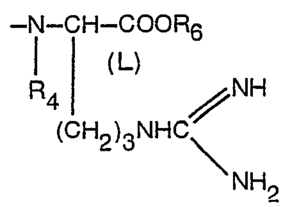
;

wherein $R_4$ is hydrogen, methyl, cyclohexyl, phenyl or benzyl and $R_6$ is as defined in claims 1 or 2.

24. A compound of claim 23 wherein X is

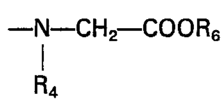

wherein $R_4$ and $R_6$ are as defined in claim 23 .

25. A compound of claim 24 wherein $R_4$ is hydrogen.

26. A compound of claim 24 wherein $R_4$ is methyl.

27. A compound of claim 24 wherein $R_4$ is phenyl.

28. A compound of claim 24 wherein $R_4$ is benzyl.

29. A compound of claim 24 wherein $R_4$ is cyclohexyl.

30. A compound of any one of the preceding claims wherein $R_3$ and $R_6$ are independently selected from hydrogen, ethyl,

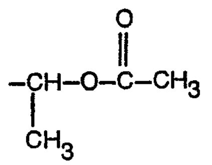 , 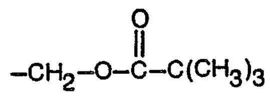 , 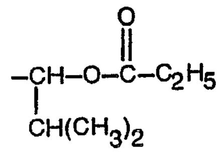 ,

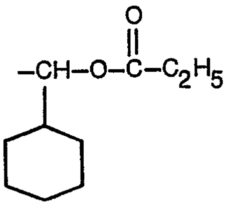 . 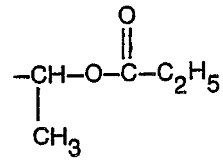 ,

and an alkali metal.

31. A compound of claim 30 wherein $R_3$ and $R_6$ are the same and are both hydrogen or an alkali metal.

32. A compound of claim 30 wherein $R_3$ is

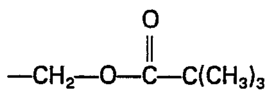

and $R_6$ is hydrogen or an alkali metal.

33. A compound of claim 30 wherein $R_3$ is ethyl and $R_6$ is hdyrogen or an alkali metal.

34. A composition for use in the treatment of hypertension comprising a pharmaceutically acceptable carrier and a compound of any one of claims 1 to 3.

35. The composition of claim 34 also including a diuretic.

**Patentansprüche**

1. Verbindung der allgemeinen Formel

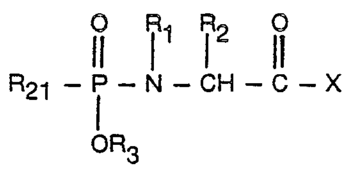

und pharmakologisch verträgliche Salze davon, in der:

X

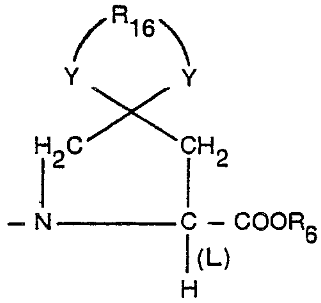

oder

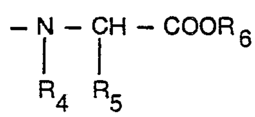 ;

bedeutet;

Y ein Sauerstoff- oder Schwefelatom darstellt;

$R_{16}$ einen Äthylen- oder Trimethylenrest bedeutet, in dem mindestens ein Kohlenstoffatomen einen Alkylsubstituenten mit 1 bis 4 Kohlenstoffatomen oder einen Dialkylsubstituenten mit 1 bis 4 Kohlenstoffatomen besitzt;

$R_4$ ein Wasserstoffatom, einen Niederalkylrest einen $-(CH_2)_m$-Cycloalkylrest oder einen Rest

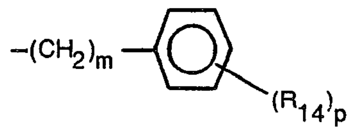

bedeutet;

$R_5$ ein Wasserstoffatomen, einen Niederalkylrest,

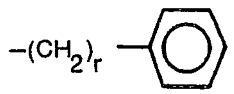 , 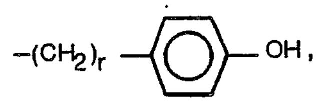

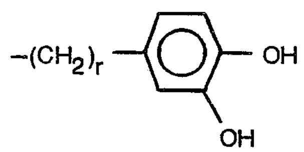 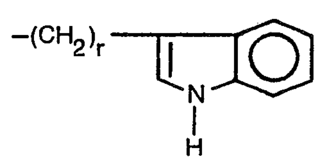 ,

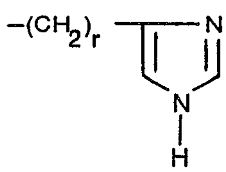 , $-(CH_2)_r-NH_2$, $-(CH_2)_r-SH$,

$-(CH_2)_r-S-$Niederalkyl, 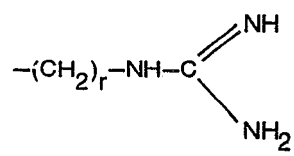 ,

oder 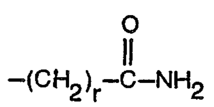 ;

bedeutet

r eine ganze Zahl im Wert von 1 bis 4 ist;

$R_1$ ein Wasserstoffatom, einen Niederalkylrest oder einen Cycloalkylrest bedeutet;

$R_2$ ein Wasserstoffatom, einen Niederalkylrest, einen halogensubstituierten Niederalkylrest,

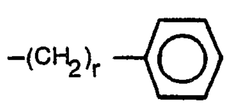 , 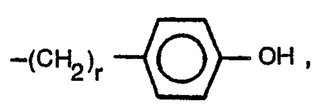 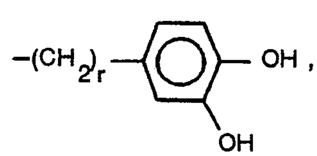

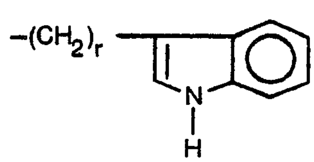 , 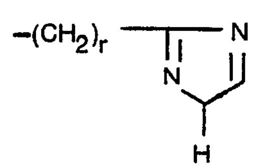 ,

$-(CH_2)_r-NH_2$ , $-(CH_2)_r-SH$ , $-(CH_2)_r-S-$Niederalkyl ,

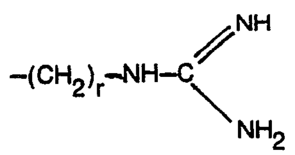 , 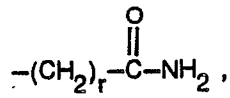 ,

darstellt; oder $R_1$ und $R_2$ zusammen eine $-(CH_2)_n$-Kette bedeuten, in der n einen Wert von 2 bis 4 aufweist;

$R_3$ und $R_6$ unabhängig voneinander ein Wasserstoffatom, ein Alkalimetall, einen Niederalkylrest, eine Benzylgruppe, eine Benzhydrylgruppe oder einen Rest

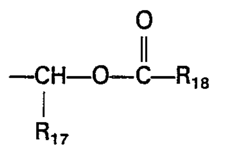

bedeuten, wobei $R_{17}$ ein Wassrstoffatom, einen Niederalkylrest, einen Cycloalkylrest oder eine Phenylgruppe darstellt und $R_{18}$ ein Wasserstoffatom, einen Niederalkylrest, einen Niederalkoxyrest oder eine Phenylgruppe bedeutet, oder $R_{17}$ und $R_{18}$ zusammen $-(CH_2)_2-$, $-(CH_2)_3$, $-CH=CH-$, oder

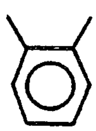

bedeuten;

$R_{21}$ einen Alkylrest mit 1 bis 10 Kohlenstoffatomen,

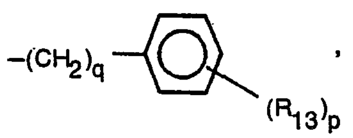 , $-(CH_2)_q-$Cycloalkyl , $-(CH_2)_s-NH_2$ ,

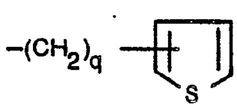 , 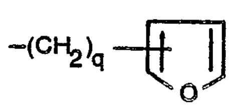 , oder 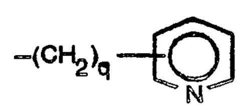

bedeutet, wobei q den Wert 0 hat oder eine ganze Zahl von 1 bis 7 bedeutet und s eine ganze Zahl im Wert von 1 bis 8 darstellt;

$R_{13}$ ein Wasserstoffatom, einen Alkylrest mit 1 bis 4 Kohlenstoffatomen, einen Alkoxyrest mit 1 bis 4 Kohlenstoffatomen, einen Alkylthiorest mit 1 bis 4 Kohlenstoffatomen, ein Chloratom, ein Bromatom, ein Fluoratom, eine Trifluormethylgruppe, eine Hydroxygruppe, eine Phenylgruppe, eine Phenoxygruppe, eine Phenylthiogruppe oder eine Phenylmethylgruppe bedeutet;

$R_{14}$ ein Wasserstoffatom, einen Niederalkylrest mit 1 bis 4 Kohlenstoffatomen, einen Alkoxyrest mit 1 bis 4 Kohlenstoffatomen, ein Chloratom, ein Bromatom, ein Fluoratom, eine Trifluormethylgruppe oder eine Hydroxylgruppe darstellt;

m den Wert 0, 1, 2 oder 3 hat; und

p den Wert 1, 2 oder 3 aufweist, mit der Maßgabe, daß p nur dann einen Wert von über 1 hat, wenn $R_{13}$ oder

$R_{14}$ ein Wasserstoffatom, eine Methylgruppe, eine Methoxygruppe, ein Chloratom oder ein Fluoratom bedeuten.

2. Verbindung nach Anspruch 1, in der

$R_4$ ein Wasserstoffatom, eine Methylgruppe, eine Cyclohexylgruppe, eine Phenylgruppe oder eine Benzylgruppe bedeutet;

$R_5$ ein Wasserstoffatom, einen Alkylrest mit 1 bis 4 Kohlenstoffatomen,

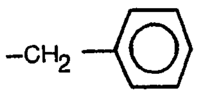 , 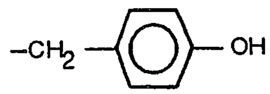 , 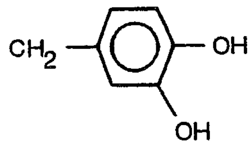 ,

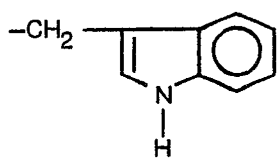 , 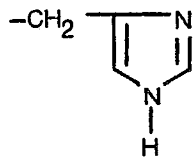 , $-(CH_2)_4-NH_2$ , $-CH_2SH$ ,

$-(CH_2)_2-S-CH_3$ , 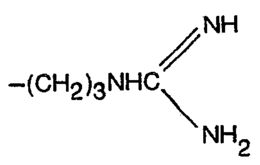 , 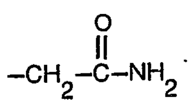 , oder 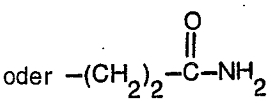 .

darstellt;

Y eine Sauerstoffatom oder ein Schwefelatom bedeutet;

$R_{16}$ einen Äthylen- oder Trimethylenrest bedeutet, in dem mindestens ein Kohlenstoffatomen einen Methyl- oder Dimethylsubstituenten besitzt;

m den Wert 0, 1 oder 2 hat;

$R_{13}$ ein Wasserstoffatom, eine Methylgruppe, eine Methoxygruppe, eine Methylthiogruppe, ein Chloratom, ein Bromatom, ein Fluoratom oder eine Hydroxylgruppe bedeutet;

$R_1$ eine Wasserstoffatom oder ein Alkylrest mit 1 bis 4 Kohlenstoffatomen darstellt;

$R_2$ ein Wasserstoffatom, einen Alkylrest mit 1 bis 4 Kohlenstoffatomen, $CF_3$ oder die Gruppe $(CH_2)_rNH_2$ bedeutet, in der r einen Wert von 1 bis 4 hat, oder

$R_1$ und $R_2$ zusammen $-(CH_2)_3-$ bedeuten;

$R_{21}$ einen Alkylrest mit 1 bis 10 Kohlenstoffatomen,

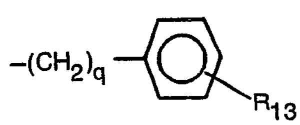

$-(CH_2)_q$-Cycloalkyl, wobei der Cycloalkylrest 5 oder 6 Kohlenstoffatomen aufweist,

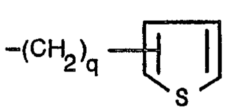 , 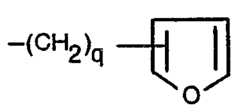 , 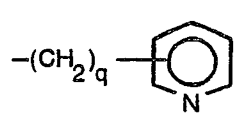 ,

oder $-(CH_2)_s-NH_2$ bedeutet, wobei q den Wert 0 hat oder eine ganze Zahl im Wert von 1 bis 4 ist, s eine ganze Zahl von 1 bis 8 ist und $R_{13}$ die vorstehend angegebene Bedeutung hat;

$R_3$ und $R_6$ unabhängig voneinander ein Wasserstoffatom eine Alkalimetall, einen Alkylrest mit 1 bis 4 Kohlenstoffatomen oder den Rest

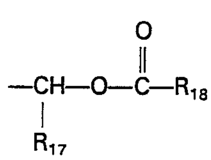

bedeuten;

$R_{17}$ ein Wasserstoffatom, eine gerade oder verzweigte Alkylgruppe mit 1 bis 4 Kohlenstoffatomen oder eine Cyclohexylgruppe bedeutet, und

$R_{18}$ eine gerade oder verzweigte Alkylkette mit 1 bis 4 Kohlenstoffatomen oder eine Phenylgruppe darstellt.

3. Verbindung nach den Ansprüchen 1 oder 2, in der $R_{21}$ ein Alkylrest mit 1 bis 10 Kohlenstoffatomen bedeutet.

4. Verbindung nach Anspruch 3, in der $R_{21}$ eine Methylgruppe bedeutet.

5. Verbindung nach Anspruch 3, in der $R_{21}$ —$(CH_2)_5$-$CH_3$ bedeutet.

6. Verbindung nach Anspruch 3, in der $R_{21}$ —$(CH_2)_7$-$CH_3$ bedeutet.

7. Verbindung nach Anspruch 1 oder 2, in der $R_{21}$ den Rest

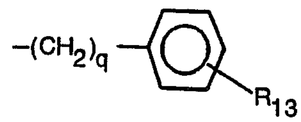

bedeutet, q den Wert 0 hat oder eine ganze Zahl von 1 bis 4 ist und $R_{13}$ ein Wasserstoffatom, eine Methylgruppe, eine Methoxygruppe, eine Methylthiogruppe, eine Chloratom, eine Bromatom, ein Fluoratom oder eine Hydroxylgruppe darstellt.

8. Verbindung nach Anspruch 7, in der $R_{21}$ ein Phenylgruppe bedeutet.

9. Verbindung nach Anspruch 7, in der $R_{21}$ eine Benzylgruppe bedeutet.

10. Verbindung nach Anspruch 7 in der $R_{21}$ eine Phenäthylgruppe bedeutet.

11. Verbindung nach Anspruch 7, in der $R_{21}$ eine 3-Phenylpropylgruppe bedeutet.

12. Verbindung nach Anspruch 7, in der $R_{21}$ eine 4-Phenylbutylgruppe bedeutet.

13. Verbindung nach Anspruch 1 oder 2, in der $R_{21}$ —$(CH_2)_s$—$NH_2$ und s eine ganze Zahl von 1 bis 8 darstellt.

14. Verbindung nach Anspruch 13, in der $R_{21}$ —$(CH_2)_6$—$NH_2$ bedeutet.

15. Verbindung nach jedem der vorangehenden Ansprüche, in der $R_1$ ein Wasserstoffatom bedeutet.

16. Verbindung nach jedem der Ansprüche 1 bis 14, in der $R_1$ eine Methylgruppe bedeutet.

17. Verbindung nach jedem der vorangehenden Ansprüche, in der $R_2$ ein Wasserstoffatom bedeutet.

18. Verbindung nach jedem der Ansprüche 1 bis 16, in der $R_2$ eine Methylgruppe bedeutet.

19. Verbindung nach jedem der Ansprüche 1 bis 16, in der $R_2$ —$(CH_2)_4$—$NH_2$ bedeutet.

20. Verbindung nach jedem der Ansprüche 1 bis 14, in der $R_1$ und $R_2$ zusammen —$(CH_2)_3$- bedeuten.

21. Verbindung nach jedem der vorangehenden Ansprüche, in der X den Rest

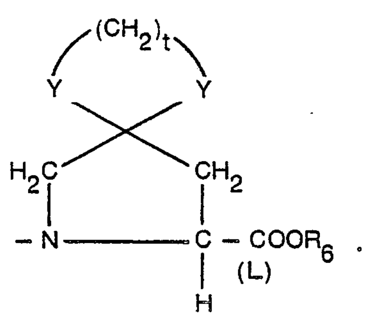

bedeutet, wobei Y ein Sauerstoff- oder Schwefelatom darstellt und t den Wert 2 oder 3 hat.

22. Verbindung nach Anspruch 21, in der X den Rest

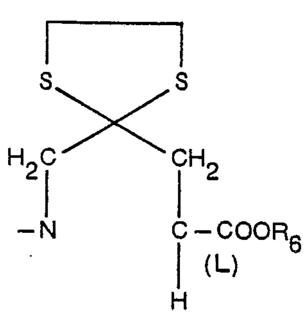

bedeutet, im dem $R_6$ die in Anspruch 1 oder 2 angegebene Bedeutung hat.

23. Verbindung nach jedem der Ansprüche 1 bis 20, in der X

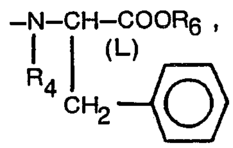 , 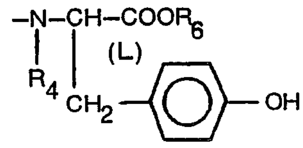

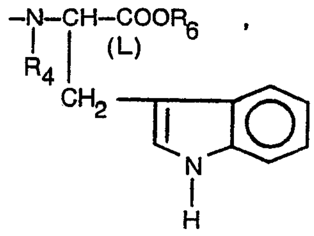 , 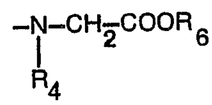 ,

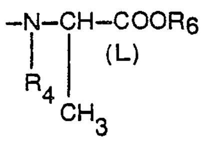 , 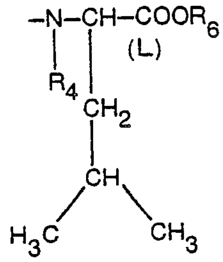 , oder 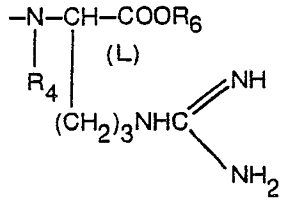

bedeutet, wobei $R_4$ eine Wasserstoffatom, eine Methylgruppe, eine Cyclohexylgruppe, eine Phenylgruppe oder eine Benzylgruppe darstellt und $R_6$ die in den Ansprüchen 1 oder 2 angegebene Bedeutung hat.

24. Verbindung nach Anspruch 23, in der X den Rest

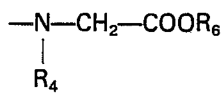

bedeutet, wobei $R_4$ und $R_6$ die in Anspruch 23 angegebene Bedeutung haben.

25. Verbindung nach Anspruch 24, in der $R_4$ ein Wasserstoffatom bedeutet.

26. Verbindung nach Anspruch 24, in der $R_4$ eine Methylgruppe bedeutet.

27. Verbindung nach Anspruch 24, in der $R_4$ eine Phenylgruppe bedeutet.

28. Verbindung nach Anspruch 24, in der $R_4$ eine Benzylgruppe bedeutet.

29. Verbindung nach Anspruch 24, in der $R_4$ eine Cyclohexylgruppe bedeutet.

30. Verbindung nach einem der vorangehenden Ansprüche, in der $R_3$ und $R_6$ unabhängig voneinander ein Wasserstoffatom, eine Äthylgruppe,

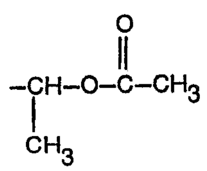 , 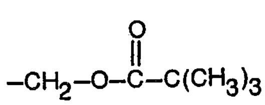 , 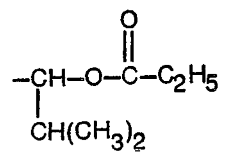 ,

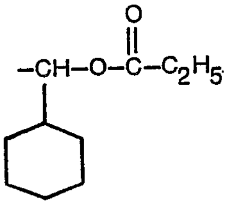 , 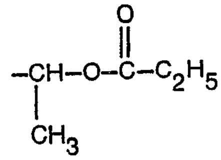 ,

oder ein Alkalimetall bedeuten.

31. Verbindung nach Anspruch 30, in der $R_3$ und $R_6$ gleich sind und jeweils ein Wasserstoffatom oder ein Alkalimetall bedeuten.

32. Verbindung nach Anspruch 30, in der $R_3$

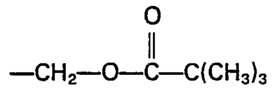

bedeutet, und $R_6$ eine Wasserstoffatom oder ein Alkalimetall ist.

33. Verbindung nach Anspruch 30, in der $R_3$ eine Äthylgruppe und $R_6$ ein Wasserstoffatom oder ein Alkalimetall bedeutet.

34. Zusammensetzung zur Verwendung bei der Behandlung von Bluthochdruck, enthaltend einen pharmakologisch verträglichen Träger une eine Verbindung nach jedem der Ansprüche 1 bis 33.

35. Verbindung nach Anspruch 34, zusätzlich ein Diuretikum enthaltend.

**Revendications**

1. Composé de formule

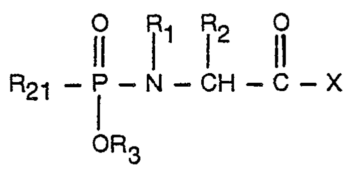

et sels pharmaceutiquement acceptables de celui-ci, formule dans laquelle

X est

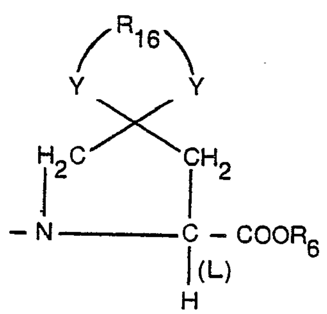

ou

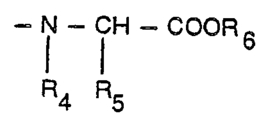

;

Y est un atome d'oxygène ou de soufre;

$R_{16}$ est un radial éthylène ou triméthylène dans lequel ou un plusieurs des atomes de carbone peuvent avoir un substituant alkyle de 1 à 4 atomes de carbone ou di(alkyle de 1 à 4 atomes de carbone);

$R_4$ est un atome d'hydrogène, ou un radical alkyle inférieur, $-(CH_2)_m$-cycloalkyle, ou

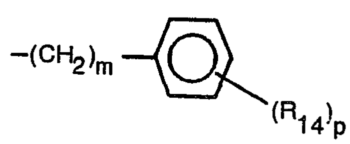

;

$R_5$ est un atome d'hydrogène, un radical alkyle inférieur, ou

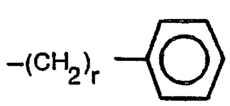

,

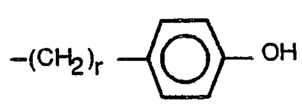

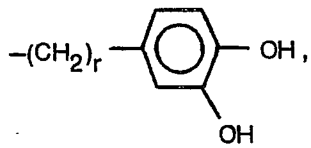

,

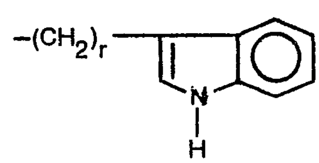

,

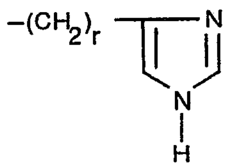 , $-(CH_2)_r-NH_2$, $-(CH_2)_r-SH$,

$-(CH_2)_r-S-$ alkyle inférieur, 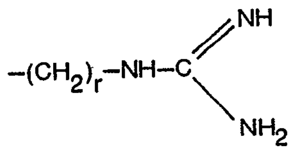 ,

ou 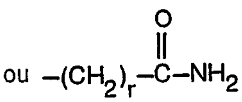 ;

r est un nombre entier de 1 à 4;

$R_1$ est un atome d'hydrogène ou un radical alkyle inférieur ou cycloalkyle;

$R_2$ est un atome d'hydrogène, un radical alkyle inférieur, alkyle inférieur halo-substitué, ou un radical de formule

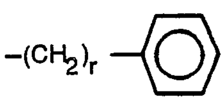 , 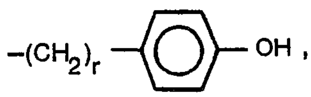 , 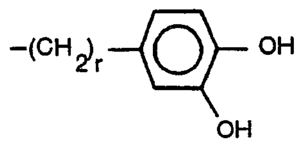

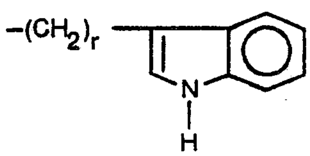 , 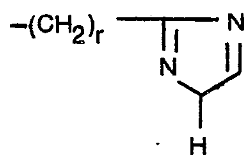 ,

$-(CH_2)_r-NH_2$, $-(CH_2)_r-SH$, $-(CH_2)_r-S-$ alkyle inférieur,

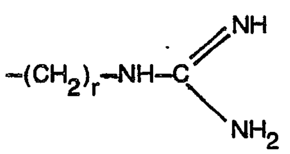 , 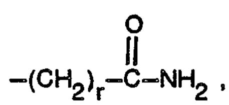 ,

ou bien $R_1$ et $R_2$ forment ensemble un groupement $-(CH_2)_n-$ dans lequel n est un nombre entier de 2 à 4;

$R_3$ et $R_6$ sont choisis indépendeamment entre les atomes d'hydrogène, les métaux alcalins et les radicaux alkyle inférieur, benzyle, benzhydryle, ou

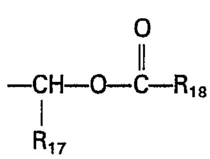

dans lequel $R_{17}$ est un atome d'hydrogène ou un radical alkyle inférieur, cycloalkyle ou phényle, et $R_{18}$ est un atome d'hydrogène ou un radical alkyle inférieur, alcoxy inférieur ou phényle, ou bien $R_{17}$ et $R_{18}$ forment ensemble un groupement de formule

$-(CH_2)_2-$, $-(CH_2)_3$, $-CH=CH-$, ou 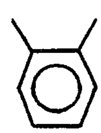 ;

$R_{21}$ est un radical alkyle de 1 à 10 atomes de carbone,

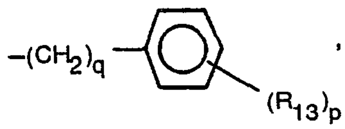 , $-(CH_2)_q-$cycloalkyle , $-(CH_2)_s-NH_2$ ,

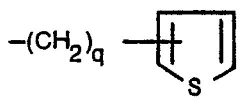 , 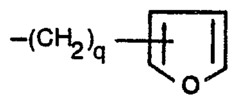 , ou 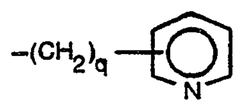

où q est égal à zéro ou est un nombre entier de 1 à 7, et s est un nombre entier de 1 à 8;

$R_{13}$ est un atome d'hydrogène, un radical alkyle de 1 à 4 atomes de carbone, alcoxy de 1 à 4 atomes de carbone, alkylthio de 1 à 4 atomes de carbone, un atome de chlore, de brome ou de fluor, ou un radical trifluorométhyle, hydroxy, phényle, phénoxy, phénylthio ou phénylméthyle;

$R_{14}$ est un atome d'hydrogène, un radial alkyle de 1 à 4 atomes de carbone, alcoxy de 1 à 4 atomes de carbone, ou alkylthio de 1 à 4 atomes de carbone, un atome de chlore, de brome ou de fluor, ou un radical trifluorométhyle ou hydroxy;

m est égal à zéro, un, deux ou trois; et

p est égal à un, deux ou trois, à condition que p ne soit plus grand que un que si $R_{13}$ ou $R_{14}$ est un atome d'hydrogène, un radical méthyle ou méthoxy, ou un atome de chlore ou de fluor.

2. Composé selon la revendication 1, dans la formule duquel:

$R_4$ est un atome d'hydrogène ou un radical méthyle, cyclohexyle, phényle ou benzyle;

$R_5$ est un atome d'hydrogène, un radical alkyle de 1 à 4 atomes de carbone, ou un radical de formule

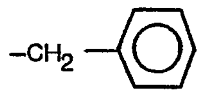 , 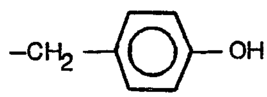 , 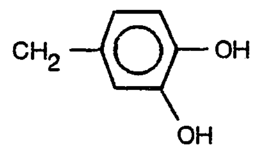 ,

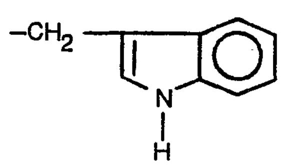 , 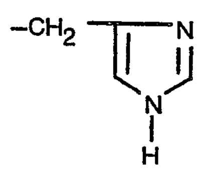 , $-(CH_2)_4-NH_2$ , $-CH_2SH$ ,

$-(CH_2)_2-S-CH_3$ , 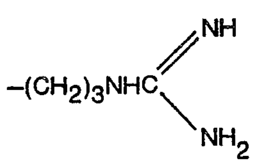 , 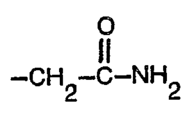 , ou 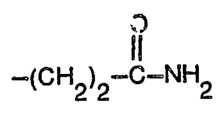 ;

Y est un atome d'oxygène ou de soufre;

$R_{16}$ est un radical éthylène ou triméthylène dans lequel ou un plusieurs des atomes a (ont) un substituant méthyle ou diméthyle;

m est métal à zéro, un ou deux;

$R_{13}$ est un atome d'hydrogène, un radical méthyle, méthoxy ou méthylthio, un atome de chlore, de brome ou de fluor, ou un groupement hydroxy;

$R_1$ est un atome d'hydrogène ou un radical alkyle de 1 à 4 atomes de carbone;

$R_2$ est un atome d'hydrogène, un radical alkyle de 1 à 4 atomes de carbone, $CF_3$, ou $-(CH_2)_rNH_2$ où r est un nombre de 1 à 4, ou bien $R_1$ et $R_2$ forment ensemble un groupement $-(CH_2)_3$-

$R_{21}$ est un radical alkyle de 1 à 10 atomes de carbone,

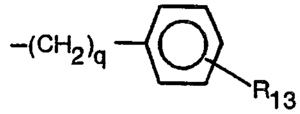

$-(CH_2)_q$-cycloalkyle où le radical cycloalkyle a 5 ou 6 atomes de carbone,

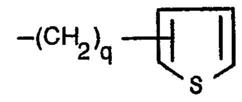 , 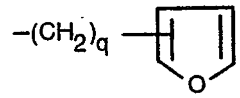 , 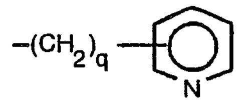 ,

ou $-(CH_2)_s-NH_2$ où q est égal à zéro ou est un nombre entier de 1 à 4, s est un nombre entier de 1 à 8, et $R_{13}$ est tel que défini ci-dessus;

$R_3$ et $R_6$ sont choisis indépendamment entre les atomes d'hydrogène et les métaux alcalins, les radicaux alkyle de 1 à 4 atomes de carbone, ou les radicaux de formule

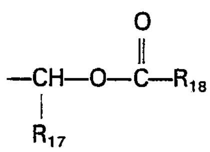

$R_{17}$ est un atome d'hydrogène, un radical alkyle à chaîne droite ou ramifiée de 1 à 4 atomes de carbone, ou un radical cyclohexyle; et $R_{18}$ est un radical alkyle à chaîne droite ou ramifiée de 1 à 4 atomes de carbone ou un radical phényle.

3. Composé selon la revendication 1 ou 2, dans la formule duquel $R_{21}$ est un radical alkyle de 1 à 10 atomes de carbone.

4. Composé selon la revendication 3, dans la formule duquel $R_{21}$ est un radical méthyle.

5. Composé selon la revendication 3, dans la formule duquel $R_{21}$ est un radical de formule $-(CH_2)_5-CH_3$.

6. Composé selon la revendication 3, dans la formule duquel $R_{21}$ est un radical de formule $-(CH_2)_7-CH_3$.

7. Composé selon la revendication 1 ou 2, dans la formule duquel $R_{21}$ est un radical de formule

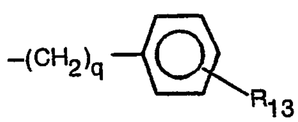

q est égal à zéro ou est un nombre entier de 1 à 4, et $R_{13}$ est un atome d'hydrogène, un radical méthyle, méthoxy ou méthylthio, un atome de chlore, de brome ou de fluor, ou un groupement hydroxy.

8. Composé selon la revendication 7, dans la formule duquel $R_{21}$ est un radical phényle.

9. Composé selon la revendication 7, dans la formule duquel $R_{21}$ est un radical benzyle.

10. Composé selon la revendication 7, dans la formule duquel $R_{21}$ est un radical phénéthyle.

11. Composé selon la revendication 7, dans la formule duquel $R_{21}$ est un radical 3-phénylpropyle.

12. Composé selon la revendication 7, dans la formule duquel $R_{21}$ est un radical 4-phénylbutyle.

13. Composé selon la revendication 1 ou 2, dans la formule duquel $R_{21}$ est un radical de formule $(-CH_2)_s-NH_2$ et s est un nombre entier de 1 à 8.

14. Composé selon la revendication 13, dans la formule duquel $R_{21}$ est un radical de formule $-(CH_2)_6-NH_2$.

15. Composé selon l'une quelconque des revendications précédentes, dans la formule duquel $R_1$ est un atome d'hydrogène.

16. Composé selon l'une quelconque des revendications 1 à 14, dans la formule duquel $R_1$ est un radical méthyle.

17. Composé selon l'une quelconque des revendications précédentes, dans la formule duquel $R_2$ est un atome d'hydrogène.

18. Composé selon l'une quelconque des revendications 1 à 16, dans la formule duquel $R_2$ est un radical méthyle.

19. Composé selon l'une quelconque des revendications 1 à 16, dans la formule duquel $R_2$ est un radical de formule $-(CH_2)_4-NH_2$.

20. Composé selon l'une quelconque des revendications 1 à 14, dans la formule duquel $R_1$ et $R_2$ forment ensemble un groupement de formule $-(CH_2)_3$.

21. Composé selon l'une quelconque des revendications précédentes, dans la formule duquel X est

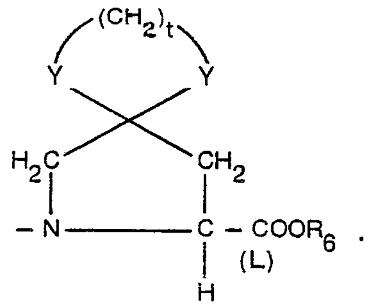

où Y est un atome d'oxygène ou de soufre, et t est égal à deux ou trois.

22. Composé selon la revendication 21, dans la formule duquel X est

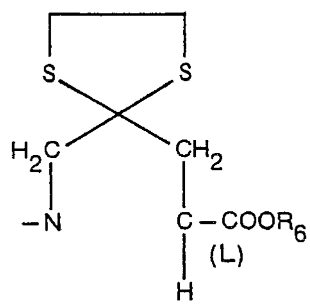

où $R_6$ est tel que défini dans la revendication 1 ou 2.

23. Composé selon l'une quelconque des revendications 1 à 20, dans la formule duquel X est

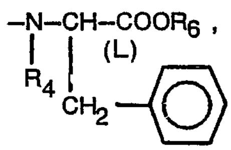,
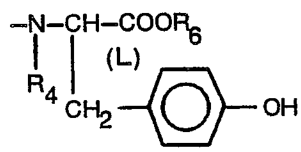

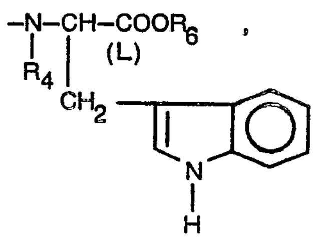,
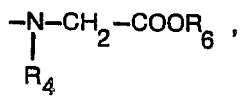,

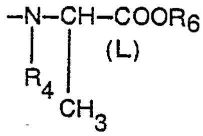,
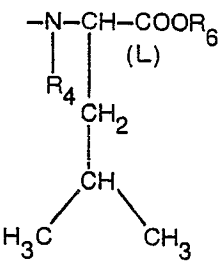, ou
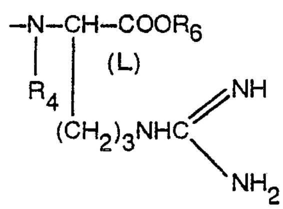;

où $R_4$ est un atome d'hydrogène ou un radical méthyle, cyclohexyle, phényle ou benzyle et $R_6$ est tel que défini dans la revendication 1 ou 2.

24. Composé selon la revendication 23, dans la formule duquel X est un radical de formule

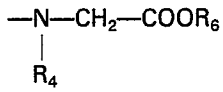

dans laquelle $R_4$ et $R_6$ sont tels que définis dans la revendication 23.

25. Composé selon la revendication 24, dans la formule duquel $R_4$ est un atome d'hydrogène.

26. Composé selon la revendication 24, dans la formule duquel $R_4$ est un radical méthyle.

27. Composé selon la revendication 24, dans la formule duquel $R_4$ est un radical phényle.

28. Composé selon la revendication 24, dans la formule duquel $R_4$ est un radical benzyle.

29. Composé selon la revendication 24, dans la formule duquel $R_4$ est un radical cyclohexyle.

30. Composé selon l'une quelconque des revendications précédentes, dans la formule duquel $R_3$ et $R_6$ sont choisis indépendamment entre l'atome d'hydrogène, le radical éthyle, les radicaux de formule

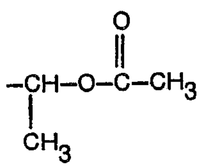 , 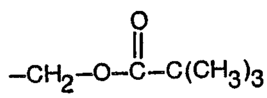 , 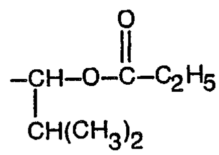 ,

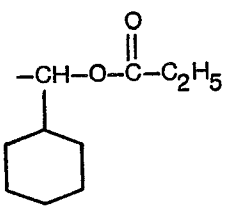 , 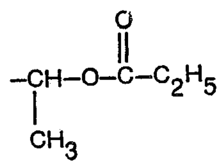 ,

et un atome de métal alcalin.

31. Composé selon la revendication 30, dans la formule duquel $R_3$ et $R_6$ sont identiques et sont tous deux un atome d'hydrogène ou de métal alcalin.

32. Composé selon la revendication 30, dans la formule duquel $R_3$ est un radical de formule

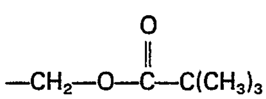

et $R_6$ est un atome d'hydrogène ou de métal alcalin.

33. Composé selon la revendication 30, dans la formule duquel $R_3$ est un radical éthyle et $R_6$ est un atome d'hydrogène ou de métal alcalin.

34. Composition destinée à être utilisée pour le traitement de l'hypertension, comprenant un porteur pharmaceutiquement acceptable et un composé selon l'une quelconque des revendications 1 à 33.

35. Composition selon la revendication 34, comportant également un diurétique.